# EUROPÄISCHE PATENTSCHRIFT

(11) **EP 1 996 694 B1**
(45) Veröffentlichungstag und Bekanntmachung des Hinweises auf die Patenterteilung: **24.02.2016**
(21) Anmeldenummer: 06829576.5
(22) Anmeldetag: 13.12.2006
(51) Int. Cl.: C12M 1/42, C12M 1/12, C12M 1/00

(54) **VORRICHTUNG UND VERFAHREN ZUR KULTIVIERUNG UND GENERIERUNG VON BIOLOGISCHEM MATERIAL IN EINEM NÄHRSTOFFNEBEL**
DEVICE AND METHOD FOR THE CULTIVATION AND PRODUCTION OF BIOLOGICAL MATERIAL IN A NUTRIENT MIST
DISPOSITIF ET PROCEDE DE CULTURE ET DE PRODUCTION DE MATIERE BIOLOGIQUE DANS UN BROUILLARD NUTRITIF

(30) Priorität: 14.12.2005 DE 102005061371
(43) Veröffentlichungstag der Anmeldung: 03.12.2008
(73) Patentinhaber: Eberhard-Karls-Universität Tübingen Universitätsklinikum, 72076 Tübingen (DE)
(72) Erfinder: SCHMIDT, Timo, 70193 Stuttgart (DE); JUST, Lothar, 72379 Stuttgart (DE)
(74) Vertreter: Witte, Weller & Partner Patentanwälte mbB
(86) Internationale Anmeldenummer: PCT/EP2006/012002
(87) Internationale Veröffentlichungsnummer: WO 2007/068467

(56) Entgegenhaltungen:
- EP-A1- 1 380 640
- EP-A1- 1 462 515
- EP-A1- 1 464 696
- EP-A2- 0 234 868
- WO-A-91/00915
- WO-A2-01/83102
- GB-A- 2 403 729
- US-A- 4 332 105
- US-A1- 2004 042 936
- DAILEY L A ET AL: "Modified polyethylenimines as non viral gene delivery systems for aerosol therapy: effects of nebulization on cellular uptake and transfection efficiency." JOURNAL OF CONTROLLED RELEASE : OFFICIAL JOURNAL OF THE CONTROLLED RELEASE SOCIETY 10 DEC 2004, Bd. 100, Nr. 3, 10. Dezember 2004 (2004-12-10), Seiten 425-436, XP002430082 ISSN: 0168-3659

## Beschreibung

Die vorliegende Erfindung betrifft eine Vorrichtung zum Kultivieren und Generieren von biologischem Material sowie ein entsprechendes Verfahren.

Im Stand der Technik sind zahlreiche Verfahren und Systeme zur Kultivierung von biologischen Zellen, Zellverbänden und Geweben bekannt. Bei diesen bekannten Verfahren bzw. Systemen werden die biologischen Zellen in der Regel mit flüssigem Kulturmedium vollständig überschichtet und für eine gewünschte Zeitspanne unter definierten Bedingungen, d.h. bei einer bestimmten Temperatur und in einer festgelegten Atmosphäre, inkubiert.

Dokument WO 91/00915 offenbart eine Vorrichtung, die einen Zerstäuber aufweist, in dem sich eine Suspension befindet, welche in ein Aerosol überführt und anschließend in eine Kammer eingeleitet wird.

Dokument US 4,332,105 offenbart eine Vorrichtung zum Besprühen von Pflanzen mit einem Mediumnebel.

Dailey et al. (2004), Modified polyethylenimines as non viral gene delivery systems for aerosol therapy: Effects of nebulization on cellular uptake and transfection efficiency, Journal of Controlled Release 100, S. 425-436, beschreiben eine Untersuchung über die Auswirkung der Zerstäubung von Komplexen aus DNA und Polyethylenimin (PEI) unter Verwendung eines Ultraschallgerätes mit der Bezeichnung Optineb.

Diese bekannten Kultivierungssysteme haben jedoch eine Vielzahl von Nachteilen. Die bekannten Systeme sind in der Regel auf die Kultivierung von zweidimensionalen biologischen Zellverbänden, sogenannten Monolayerkulturen ausgerichtet. Die Kultivierung von dreidimensionalen Gewebekonstrukten, Organen , Embryonen etc. ist mit den bekannten Systemen hingegen nur bedingt realisierbar, insbesondere, wenn dies im Großmaßstab erfolgen soll. So wird bei den bekannten Systemen bspw. die Gasversorgung der einzelnen biologischen Zellen des dreidimensionalen Gewebekonstruktes wenig zufriedenstellend bzw. nur mit sehr großem technischen und finanziellen Aufwand erreicht. Bei einigen dieser Systeme, den sog. Roller-Kulturen, treten außerdem Scherkräfte auf, die negative Nebeneffekte auf das Zellverhalten induzieren. Weiter steigt bei den bekannten Kultursystemen mit der Größe und Komplexität der Letzteren bekanntermaßen die Gefahr von bakteriellen Verunreinigungen.

Ferner weisen die bekannten Systeme bezüglich der heutigen Anforderungen moderner Zellkulturtechnologien Limitationen auf. Die Mehrzahl der Systeme aus dem Stand der Technik sind im Wesentlichen auf die reine Kultivierung von biologischen Zellen und Geweben ausgerichtet, d.h. dem bloßen "am Leben halten" *in vitro.* Sie sind nur bedingt im Rahmen der Neubildung bzw. Generierung von biologischen Gewebekonstrukten oder gar ganzen Organen mit Mitteln des sog. "Tissue Engineerings" geeignet. Unter Tissue Engineering versteht man gemäß der Definition der USamerikanischen National Science Foundation (NSF) aus dem Jahre 1988 die Anwendung von Prinzipien und Methoden der Ingenieurs- und Lebenswissenschaften für das grundlegende Verständnis der Wechselwirkung von Struktur und Funktion normalen und kranken Gewebes sowie zur Entwicklung von biologischem Gewebeersatz zur Rekonstruktion, dem Erhalt oder der Verbesserung der Gewebefunktionen. Dabei beruht ein Prinzip des Tissue Engineerings darauf, spezifische Zellen außerhalb des Organismus in der Zellkultur, d.h. *in vitro,* zu vermehren und zielgerichtet im Gewebeverband zu differenzieren und dadurch biologische Gewebekonstrukte mit gewünschten Eigenschaften zu generieren. Ein Ziel des Tissue Engineerings ist es, geschädigtes Gewebe in einem Organismus durch *in vitro* generierte Gewebekonstrukte mit oder ohne Hilfskonstrukte, wie Matrices, zu ersetzen und damit die durch die Zellschädigung verursachten Funktionsverluste aufzuheben.

Vor diesem Hintergrund ist es Aufgabe der vorliegenden Erfindung, eine Vorrichtung und ein Verfahren sowohl zur Kultivierung als auch zur Generierung von biologischem Material bereitzustellen, bei dem die vorstehend genannten Nachteile aus dem Stand der Technik vermieden werden. Insbesondere soll diese Vorrichtung und dieses Verfahren eine optimierte Kultivierung von dreidimensionalen biologischen Gewebekonstrukten sowie die Generierung und Neuerzeugung von strukturellen biologischen Systemen, wie Geweben, Organen etc., bspw. im Rahmen des Tissue Engineerings, ermöglichen.

Die der Erfindung zugrunde liegende Aufgabe wird gelöst durch eine Vorrichtung zur Kultivierung und Generierung von biologischem Material mit den Merkmalen des Anspruchs 1

Die der Erfindung zugrunde liegende Aufgabe wird ferner gelöst durch ein Verfahren zum Kultivieren und Generieren von biologischem Material mit den Merkmalen des Anspruchs 13.

Dabei wird erfindungsgemäß unter "Kultivieren" von biologischem Material verstanden, dass Letzteres nach Herauslösung aus seiner natürlichen oder ursprünglichen Umgebung *in vitro* gehalten und - wahlweise - vermehrt und in seinen Eigenschaften verändert wird.

Hierzu im Gegensatz wird unter "Generieren" erfindungsgemäß verstanden, dass ausgehend von biologischem Ausgangsmaterial mit bestimmten Ausgangseigenschaften biologisches Zielmaterial mit gewünschten Zieleigenschaften neu und zielgerichtet gebildet wird. "Generieren" bedeutet erfindungsgemäß also das gezielte Neuerzeugen von biologischen Strukturen bzw. von strukturellen biologischen Systemen. Bei der Generierung des Zielmaterials können die Techniken des Tissue Engineerings Anwendung finden, mit denen die Differenzierung des biologischen Materials, z.B. durch Beeinflussung der Genexpression, gesteuert wird. Durch die erfindungsgemäße Generierung lassen sich bspw. aus Epithelzellen Hautkonstrukte oder aus Knorpelzellen dreidimensionale Knorpelersatzstrukturen bilden, die zu pharmakologischen oder auch Therapiezwecken verwendet werden können.

Erfindungsgemäß kommen als "biologisches Material" sämtliche beliebig undifferenzierte Stamm- und Vorläuferzellen oder differenzierte Zellen tierischen, humanen, pflanzlichen, bakteriellen Ursprungs, Verbände aus vorgenannten Zellen, Organe, Transplantate, Embryonen, Organismen etc. in Frage. Das erfindungsgemäße biologische Material kann außerdem mit molekularbiologischen Methoden genetisch manipuliert sein. Ausgenommen sind jedoch menschliche Lebewesen, menschliche Embryonen und menschliche embryonale Stammzellen.

Als "Einrichtung zur Erzeugung von Ultraschallwellen" kann ein Ultraschallgenerator vorgesehen sein, der Keramiken oder Halbleiterpolymere als Schwingungsplatten aufweisen kann. Die Schwingungsplatten des Ultraschallgenerators lassen sich mit Frequenzen im Ultraschallbereich in Schwingungen bzw. Vibration versetzen und können mit dem Ultraschallwellen transmittierenden Medium in Kontakt gebracht werden. Diese Schwingungen können in dem Ultraschallwellen transmittierenden Medium eine Wellenbewegung verursachen. Der Ultraschallgenerator ist derart angeordnet, dass sich dieser direkt in das Ultraschallwellen transmittierenden Medium einbringen lässt, wobei es jedoch ausreichend ist, wenn das ultraschallwellenerzeugende Element mit dem Ultraschallwellen transmittierenden Medium in Kontakt steht. Ultraschallgeneratoren sind im Stand der Technik allgemein bekannt. Beispielhaft wird auf die EP 0 234 868 B1, JP 59 045 879 A und US 4,332,105 verwiesen.

Als "Kammer" kommt jedes beliebige Behältnis in Frage, das zur Aufnahme, Kultivierung, Generierung des biologischen Gewebes und zur Aufnahme eines Kulturmediums geeignet bzw. ausreichend Raum aufweist und - vorzugsweise - gegenüber der Umgebung abgedichtet werden kann. Die Kammer muss ferner in der Lage sein einen Aerosolnebel aus zerstäubtem Kulturmedium zu fassen. Dabei ist es in das Belieben des Fachmanns gestellt neben einer sogenannten "Nebelkammer", die das Kulturmedium in flüssiger Form fassen kann und zur Erzeugung des Aerosolnebels aus zerstäubtem Kulturmedium dient, eine oder mehrere weitere Kammern vorzusehen, bspw. "Kulturkammern", die das biologische Material fassen können und mit der "Nebelkammer" in Verbindung stehen. Die Kammer kann ferner in einzelne Nebenräume unterteilt sein, wovon einer die Funktion einer "Nebelkammer" und ein weiterer die Funktion einer "Kulturkammer" erfüllt. Derartige Kammern sind in Zellkulturlaboren üblich und dem Fachmann, bspw. einem Zellbiologen, bekannt. Eine Kammer ist bspw. ein durch Wände, eine Decke und einen Boden abgegrenzter Raum.

Als "Kulturmedium" kommt jedes im Bereich der Zellkultivierung bekannte Medium in Fragen, wie beispielsweise DMEM Low Glucose, DMEM high Glucose, DMEM/Ham's F-12, Ham's F-10, Ham's F-12, Iscove's modified DMEM, L-15 Medium (Leibovitz's), Medium 199 mit Earle's Salzen, Medium 199 mit Hank's Salzen, Minimal Essential Medium Alpha Modification, PMI 1640, DMEM READY MIX, RPMI 1640 READY MIX, Thermostable Media; alle erhältlich bei PAA Laboratories GmbH, Pasching, Österreich.

Unter einem "Ultraschallwellen transmittierenden Element" wird ein solches Element verstanden, das Ultraschallwellen an das umgebende Medium weiter leiten kann. Das Ultraschallwellen transmittierenden Element weist dazu eine Materialdichte auf, die diesem vorzugsweise ähnliche oder höchst bevorzugt dieselben akustischen Eigenschaften wie die des umgebenden Mediums verleiht. Die Eigenfrequenzen des Elementes und des umgebenden Mediums sind vorzugsweise ähnlich und höchst bevorzugt identisch. Weist das umgebende Medium eine hohe Dichte auf, wird vorzugsweise ein Element mit einer ebenfalls hohen Dichte eingesetzt, weist das umgebende Medium eine niedrige Dichte auf, wird vorzugsweise ein Element mit einer niedrigen Dichte eingesetzt. Erfindungsgemäß weist das Ultraschallwellen transmittierenden Element zumindest zwei Seiten bzw. Oberflächen auf, die derart ausgerichtet sind, dass die erste Oberfläche mit dem Kulturmedium und die zweite Oberfläche mit dem Ultraschallwellen transmittierenden Medium in Kontakt treten kann.

Ein "Ultraschallwellen transmittierendes Medium" ist ein solches Medium, in dem durch Beaufschlagung mit Ultraschall Wellen erzeugt und letztere an die Umgebung weitergeleitet werden können. Dabei ist es bevorzugt, wenn das Medium die Ultraschallwellen mit möglichst geringem Verlust an die Umgebung weiterleitet. Geeignete Medien sind bspw. Flüssigkeiten oder Gele, die sich in ihren Eigenschaften im Hinblick auf die Schallübertragung, bspw. über ihre spezifische Dichte, beliebig auswählen lassen.

Erfindungsgemäß ist die Einrichtung zur Erzeugung von Ultraschallwellen, bspw. der Ultraschallgenerator, außerhalb der Kammer und damit außerhalb des Kulturmediums angeordnet. Dies hat den Vorteil, dass Kontaminationen des Kulturmediums des biologischen Materials durch den Ultraschallgenerator vermieden und in der Kammer ggf. unter sterilen Bedingungen gearbeitet werden kann. Ferner wird eine schädigende Wärmeübertragung durch den Ultraschallgenerator auf das Kulturmedium und damit das biologische Material weitgehend vermieden. Die für die jeweilige Kultivierung bzw. Generierung des biologischen Materials erforderliche Temperatur lässt sich dadurch besonders gut kontrollieren.

Die Weiterleitung der Ultraschallwellen in das Innere der Kammer und das dortige Kulturmedium erfolgt mittels des Ultraschallwellen transmittierenden Mediums und des Ultraschallwellen transmittierenden Elementes. Die Ultraschallwellen, die in das Ultraschallwellen transmittierende Medium eingeleitet werden, treffen auf die mit dem letzteren in Kontakt stehende zweite Oberfläche des Ultraschallwellen transmittierende Elementes. Dadurch wird das Ultraschallwellen transmittierende Element angeregt und leitet die Schallwellen über die erste Oberfläche, die mit dem Kulturmedium in Kontakt steht, in letzteres weiter.

Durch die Weiterleitung der Ultraschallwellen in das Kulturmedium entstehen "Tröpfchen" des Kulturmediums in der Kammer in Form eines Dampfes, Aerosols, Nebels oder einer Zerstäubung. Die Tröpfchen haben dabei etwa einen Durchmesser, der bei ca. 0,01 bis 10 µm liegt, jedoch auch größer oder kleiner sein kann. Die mittels eines solchen Verneblungsverfahrens erzeugten Tröpfchen werden in der Kammer vorzugsweise gleichmäßig verteilt.

Die der Erfindung zugrunde liegende Aufgabe wird damit vollkommen gelöst. Die Erfinder stellen erstmalig ein Verfahren zur Generierung von biologischem Material bereit, das die eingangs genannten Probleme aus dem Stand der Technik vermeidet, und eröffnen damit vollkommen neue Möglichkeiten für die Zellkulturtechnik.

So ist insbesondere im Hinblick auf die mittels Tissue Engineerings zu generierenden dreidimensionalen biologischen Materialien oder auch solchen flächigen biologischen Materialien, bei denen mindestens ein Krümmungsradius nicht unendlich ist, das Bereitstellen des Kulturmediums in Form eines aus Tröpfchen bestehenden Mediumsdampf einem herkömmlichen Kultursystem, bei dem das biologische Material mit Medium überschichtet wird, in mehreren Punkten überlegen. Durch die Mediumsbedampfung des Materials ergibt sich eine bessere Gasdiffusion im Gewebe und damit bspw. eine verbesserte Sauerstoffversorgung. Dreidimensionale Gewebekonstrukte haben meist einen geringeren Oberflächenanteil im Verhältnis zum beanspruchten Volumen, was bei einer normalen Kultivierung durch Überschichtung mit Medium zu einer ungünstigen Sauerstoffverfügbarkeit führt. Des Weiteren ist von Vorteil, dass das erfindungsgemäße Verfahren durch die Bereitstellung von Tröpfchen weniger Nährmedium verbraucht als herkömmliche Kultivierungsverfahren. Ferner wird durch die Mediumsbedampfung eine Verunreinigung des Kulturmediums mit Bakterien minimiert, da diese bei der Zerstäubung geschädigt oder sogar getötet werden. Ein weiterer Vorteil ist, dass keine Scherkräfte auftreten, die bspw. bei den Roller-Kulturen bekannt sind. Eine limitierte Größe des zu generierenden Gewebekonstruktes, wie diese bei herkömmlichen Kultivierungstechniken durch diverse Anforderungen an das System auftreten, ist nicht gegeben. Die Erfinder haben ferner erkannt, dass Transplantate, die im Rahmen des erfindungsgemäßen Verfahrens kultiviert oder generiert werden, auch über einen längen Kulturzeitraum ihre Funktionalität beibehalten.

Bei der Vorrichtung kann die Einrichtung zur Erzeugung von Ultraschallwellen derart ausgestaltet sein, dass diese einen Ultraschallbrennpunkt bilden kann. Bei dem erfindungsgemäßen Verfahren ist es bevorzugt, wenn in Schritt (b) die Ultraschallwellen derart in das transmittierende Medium eingeleitet werden, dass ein Ultraschallbrennpunkt in dem Kulturmedium oder in dem Ultraschallwellen transmittierenden Medium entsteht.

Diese Maßnahmen haben den Vorteil, dass sich das Ausmaß der Tröpfchenbildung in dem Kulturmedium erleichtert kontrollieren lässt. Im Ultraschallbrennpunkt werden die Ultraschallwellen fokussiert, welcher die energiereichste Stelle darstellt.

In der erfindungsgemäßen Vorrichtung kann die Einrichtung zur Erzeugung von Ultraschallwellen derart ausgestaltet sein, dass diese den Ultraschallbrennpunkt in dem Kulturmedium positionieren, weiter vorzugsweise in seiner Position in dem Kulturmedium verändern, und höchst bevorzugt in seiner Position in dem Kulturmedium derart verändern kann, dass dieser mit der Oberfläche des Kulturmediums zusammen fällt. In einer bevorzugten Weiterbildung des erfindungsgemäßen Verfahrens ist es bevorzugt, wenn in Schritt (b) die Ultraschallwellen derart in das Kulturmedium eingeleitet werden, dass der Ultraschallbrennpunkt mit der Oberfläche des Kulturmediums zusammen fällt.

Eine optimale Vernebelung des Kulturmediums hängt entscheidend von der Position des Ultraschallbrennpunktes im Kulturmedium ab. Durch die Möglichkeit der flexiblen Positionierung und Ortsveränderung des Ultraschallbrennpunktes in dem Kulturmedium lässt sich die Intensität und das Ausmaß der Nebelbildung in der Kammer beliebig regulieren. Durch die Verschiebung, die vorzugsweise in vertikaler aber auch in horizontaler Richtung erfolgen kann, lassen sich Temperaturspitzen in dem Medium vermeiden, das sich im Bereich der energiereichsten Stelle das Kulturmedium am stärksten erwärmt. Liegt der Ultraschallbrennpunkt direkt an der Oberfläche des Kulturmediums, erreicht man eine maximale Zerstäubung.

Das Ultraschallwellen transmittierende Element ist vorzugsweise in dem Boden der Kammer angeordnet.

Durch diese Maßnahme werden die konstruktiven Voraussetzungen für eine optimale Anordnung des Ultraschallwellen transmittierenden Elementes geschaffen. Dabei ist das Element bspw. in ein Fenster, das im Boden der Kammer vorgesehen ist, eingesetzt, beispielsweise durch Pressen, Verschrauben, Verschweißen, Verkleben oder Verklemmen. Weiter kann das Element auch den gesamten Boden oder Kammer bilden. Dabei wird sichergestellt, dass der Innenraum der Kammer gegenüber dem Außenraum der Kammer flüssigkeits- und vorzugsweise luftdicht abgetrennt ist, so dass kein Ultraschallwellen transmittierendes Medium in die Kammer bzw. Kulturmedium aus der Kammer gelangen bzw. treten kann.

Erfindungsgemäß ist es bevorzugt, wenn das Ultraschallwellen transmittierende Element eine Platte, Membran oder eine Folie ist, und aus einem Material besteht, das Ultraschallwellen optimal transmittiert. Beispiele für geeignete Materialien sind: Keramiken, Glas, Mineralien, Metalle, Metalllegierungen und Kunststoffe, wobei die Platte oder Membran vorzugsweise eine Dicke aufweist, die zwischen 0,005 mm und 1 cm, und höchst bevorzugt bei 0,025 mm liegt.

Letztere Ausgestaltungen und Materialien eignen sich aufgrund ihrer akustischen Eigenschaften besonders gut, um die außerhalb der Kammer erzeugten Ultraschallwellen in das Kulturmedium weiterzuleiten.

Die transmittierenden Eigenschaften des Elementes lassen sich verbessern, indem die Platte oder die Membran konvex in Richtung des Ultraschallwellen transmittierenden Mediums gewölbt ist.

Die transmittierenden Eigenschaften des Elementes sind besonders gut, wenn dieses im Wesentlichen die gleiche Dichte und die gleichen Ultraschalltransmissionseigenschaften aufweist wie das Kulturmedium und/oder das Ultraschallwellen transmittierende Medium.

Bei dieser Voraussetzung erfolgt die Weiterleitung der Ultraschallwellen von dem außerhalb der Kammer angeordneten Medium in das Kulturmedium im Inneren der Kammer mit minimalem Verlust und es kann eine optimale Zerstäubung des Kulturmediums erzielt werden.

Nach einer bevorzugten Ausgestaltung weit die Vorrichtung ferner eine das Ultraschallwellen transmittierende Medium aufnahmefähige zweite Kammer auf, wobei diese weiter bevorzugt eine begrenzende Wand aufweist, die wiederum das Ultraschallwellen transmittierende Element aufweist.

Mit dieser Maßnahme werden die konstruktiven Vorraussetzungen für eine erfindungsgemäße Vorrichtung auf vorteilhafte Weise gelöst. Die zweite Kammer, die auch als Transmissionskammer bezeichnet werden kann, kann direkt an den Boden der Kultivierungskammer angeordnet werden, so dass der Boden der letzteren gleichzeitig die Decke der zweiten Kammer darstellt. Der Boden der Kultivierungskammer stellt damit die die zweite Kammer begrenzende Wand bereit. Dadurch wird auf kostengünstige Weise eine zusammenhängende Vorrichtungseinheit bereitgestellt. Die zweite Kammer ist vorzugsweise derart ausgestaltet, dass diese zur zumindest teilweisen Aufnahme der Einrichtung zur Erzeugung von Ultraschallwellen fähig ist.

Diese Ausgestaltung ermöglicht es, dass der Ultraschallgenerator entweder direkt oder aber sein Ultraschallwellen generierendes Element in das Ultraschallwellen transmittierende Medium eingebracht werden kann. Eine teilweise Aufnahme kann dadurch realisiert werden, dass die zweite Kammer eine Öffnung aufweist, die zum Einsetzen eines bspw. stabförmigen Elementes geeignet ist, an dessen Ende sich das Ultraschallwellen generierende Element befindet.

Die erfindungsgemäße Vorrichtung weist ferner eine Einrichtung zur Erzeugung von auf das biologische Material einwirkenden physiologischen oder/und physikalischen Stimuli auf. Bei dem erfindungsgemäßen Verfahren ist es bevorzugt, wenn in einem weiteren Schritt (c) eine physiologische oder/und physikalische Stimulierung des biologischen Materials erfolgt.

Erfindungsgemäß wird unter "physiologischer Stimulierung" die Induktion von Differenzierungs-, Proliferations- oder molekularen Entwicklungsprozessen in dem biologischen Material durch direkte oder indirekte Wechselwirkung mit biologischen, chemischen und physikalischen Faktoren verstanden. Beispiele für solche Faktoren sind Wachstums- und Kulturmediumsfaktoren, biologische und nicht-biologische Matrices, die Ionenzusammensetzung oder der pH-Wert des Kulturmediums.

Erfindungsgemäß wird unter "physikalischer Stimulierung" die Induktion von Differenzierungs-, Proliferations- oder molekularen Entwicklungsprozessen in dem biologischen Material durch direkte oder indirekte Einwirkung von physikalischen Kräften und/oder Energien auf das Material verstanden. Beispiele für derartige Kräfte und Energien sind: Druck-, Zug-, Scher-, Zentrifugal-, Zentripedal-, Gravitations-, Mikrogravitationskraft, kinetische Energie, elektrische Impulse und Wellen, Magnetismus, Temperatur, etc.

Einrichtungen zur physiologischen und physikalischen Stimulierung umfassen mechanische, pneumatische, hydraulische Systeme, Rotationsantriebe, Generatoren, Magneten, Heizvorrichtungen, Kultivierungsvorrichtungen, natürliche Biomaterialien, künstliche Biomaterialien etc. und stehen dem Fachmann umfangreich zur Verfügung. Die einzelnen Parameter der Stimulierung, wie die Art des Stimulus, die Dauer und das Muster der Stimulierung sind dem Fachmann überlassen und sind bspw. abhängig von der Art des biologischen Ausgangs- und Zielmaterials, dem späteren Einsatzzweck des generierten biologischen Materials, evtl. während der Generierung durchzuführender Experimente an dem biologischen Material etc.

Die Erfinder kombinieren erstmals auf erfinderische Art und Weise Erkenntnisse aus der Zellkultivierungstechnologie mit solchen aus der modernen Zellbiologie und Biotechnologie. So handelt es sich bei den Verfahren, die aus den EP 0 234 868 B1, JP 59 045 879 A und der US 4,332,105 bekannt sind, um Verfahren zur reinen Kultivierung von pflanzlichen Zellen, die keine Stimulierungseinrichtung oder vergleichbare Maßnahmen zur genetischen Manipulation von biologischem Material vorsehen und deshalb zur erfindungsgemäßen Generierung von biologischem Material, insbesondere tierischen oder humanen Ursprungs, ungeeignet sind. Besonders überraschend war es, dass Maßnahmen aus den bekannten Verfahren, wie die Erzeugung und Bereitstellung von Tröpfchen aus Kulturmedium, erfolgreich auch zur Generierung von biologischen Strukturen mittels Tissue Engineerings angewendet werden können. So konnten die Erfinder erfolgreich Hühnerembryonen bis zu einem spezifischen Entwicklungsstadium "kultivieren", wobei sämtliche gemessenen physiologischen Parameter eine normale Entwicklung während des analysierten Zeitraums bestätigten. Dies war ausgehend von den Daten aus den vorgenannten Dokumenten, die sich mit der bloßen Kultivierung von Pflanzenzellen befassen, aufgrund der biologischen Komplexität der Embryonen nicht zu erwarten.

Bei der erfindungsgemäßen Vorrichtung wird die Einrichtung zur Erzeugung von physiologischen Stimuli durch eine Vorrichtung zur Zufuhr von Wachstumsfaktoren in das Kulturmedium realisiert. Bei dem erfindungsgemäßen Verfahren kann die physiologische Stimulierung durch In-Kontakt-Bringen des biologischen Materials mit Wachstumsfaktoren realisiert werden, die vorzugsweise in dem Kulturmedium bereitgestellt werden.

Durch diese Maßnahme werden auf vorteilhafte Art und Weise die Wachstumsfaktoren als physiologische Stimuli dem biologischen Material über das Kulturmedium zugeführt. Die Zufuhrvorrichtung kann bspw. einen Aufnahmebehälter für das Kulturmedium umfassen, der vorzugsweise außerhalb der Kammer angeordnet und damit für die Zugabe der Wachstumsfaktoren leicht zugänglich ist. An den Aufnahmebehälter ist vorzugsweise ein Schlauch angeschlossen, der durch eine Peristaltikpumpe geführt werden kann und das Medium mit den darin gelösten Wachstumsfaktoren in die Kammer einbringt. Da manche Wachstumsfaktoren eine begrenzte Lebensdauer haben, können diese dem Kulturmedium beliebig oft zugegeben und in Ihrer Konzentration variierend zugegeben werden. Es versteht sich, dass weitere Zufuhrvorrichtungen, die routinemäßig in biologischen oder chemischen Laboren Verwendung finden, eingesetzt werden können.

Alternativ bzw. zusätzlich kann in der erfindungsgemäßen Vorrichtung die Einrichtung zur Erzeugung von physiologischen Stimuli durch eine mit dem biologischen Material in Kontakt stehende biologische Matrix oder/und eine nicht-biologische Matrix realisiert werden. In einer bevorzugten Weiterbildung des erfindungsgemäßen Verfahrens wird die physiologische Stimulierung durch In-Kontakt-Bringen des biologischen Materials mit einer biologischen Matrix oder/und einer nicht-biologischen Matrix realisiert.

Die Wachstumsfaktoren können auf biologische oder nicht-biologische Matrices an- bzw. in diese eingebracht werden, mit denen das biologischen Material inkubiert wird. Dabei wird sichergestellt, dass die Wachstumsfaktoren an die Oberfläche des biologischen Materials gelangen und durch Wechselwirkung mit spezifischen Oberflächenmolekülen und -rezeptoren intrazelluläre Differenzierungs- und/oder Proliferationsprozesse auslösen können.

Durch diese Maßnahme kann der Generierungsprozess besonders vorteilhaft durch die Wahl entsprechender Wachstumsfaktoren gesteuert werden. Erfindungsgemäß werden unter "Wachstumsfaktoren" allgemein Substanzen verstanden, die biologische Wachstums- und Entwicklungsprozesse kontrollieren. Beispiele für solche Wachstumsfaktoren sind: Fibroblast Growth Factor (FGF), Bone Morphogenetic Protein (BMP), Keratinocyte Growth Factor (KGF), Epidermal Growth Factor (EGF), Nerve Growth Factor (NGF), Insulin-like Growth Factor (IGF), Transforming Growth Factor (TGF), Tumor Necrosis Factor (TNF) etc. Die Wahl und Konzentration des jeweiligen Wachstumsfaktors sowie die Art und Weise der Stimulierung hängt von dem gewünschten biologischen Material ab, das es zu generieren gilt, und liegt im Belieben des Fachmannes.

Erfindungsgemäß wird gewährleistet, dass die Wachstumsfaktoren dem biologischen Material wirksam zugeführt werden und die physiologische Stimulierung des Letzteren dadurch einfach realisiert wird.

Durch die alternative physiologische Stimulierung durch In-Kontakt-Bringen des biologischen Materials mit einer biologischen Matrix oder/und einer nicht-biologischen Matrix wird sichergestellt, dass die Matrices in direktem Kontakt mit dem biologischen Material stehen und ein Komposit mit letzterem bilden. Durch diese Maßnahme werden zur Stimulierung des biologischen Materials auf vorteilhafte Art und Weise Erkenntnisse der modernen Zell- und Entwicklungsbiologie genutzt. So ist in Fachkreisen bekannt, dass die Expression und der Erhalt spezifischer Gewebeeigenschaften wesentlich von der Verankerung der Zellen und damit von der Oberfläche des Substrates während der Kultivierung abhängig ist. Die Stimulierung über Matrices nimmt somit Einfluss auf die Differenzierungs- und Proliferationsprozesse, die Form, den Halt und die Elastizität des zu generierenden biologischen Materials.

Als biologische Matrices kommen sämtliche dem Fachmann zur Verfügung stehende biogenen Substanzen in Frage, wie bspw. Kollagene, Fibrin, Chitin, Laminine, Fibronektin, Hyaluronsäure oder Hydroxyapatit. Über die Wahl der biogenen Substanzen lässt sich gezielt das zu generierende Material steuern. Mit dem erfindungsgemäßen Verfahren lässt sich bspw. über Rinderkollagen Typ I als biogene Matrixsubstanz durch In-Kontakt-Bringen mit Knorpelzellen als biologisches Ausgangsmaterial ein dreidimensionaler Knorpelersatz generieren. Als eine weitere biologische Matrix kommt die sog. extrazelluläre Matrix (ECM) oder ein zellfreies Gewebegerüst aus ECM in Frage, das über dem Fachmann bekannte Verfahren aus nativen Geweben gewonnen wird. Bei diesem Verfahren bleibt die komplexe dreidimensionale Anordnung der extrazellulären Matrixproteine erhalten und gewährleistet somit eine möglichst *in* vivo-nahe Differenzierung der biologischen Gewebe. Diese Matrices werden bereits aus Herzklappen, dem Dünndarm, der Speiseröhre und der Dermis hergestellt; vgl. Tian et al. (2005), Effect of small intestinal submucosa on islet recovery and function in vitro culture, Hepatobiliary Pancreat. Dis. Int. 4(4), Seiten 524-9; Ruszczak, Z. (2003), Effect of collagen matrices on dermal wound healing, Adv. Drug Deliv. Rev. 55(12), Seiten 1595-611; Zhu et al. (2005), The growth improvement of porcine esophageal smooth muscle cells on collagen-grafted poly(DL-Lactide-co-Glycolide) membrane, J. Biomed. Mater. Res. B. Appl. Biomater. 75(1), Seiten 193-9; Schenke-Layland et al. (2003), Complete dynamic repopulation of decellularized heart valves by application of defined physical signals - an in vitro study, Cardiovasc. Res. 60(3), Seiten 497-509. Hierbei ist von Vorteil, dass sowohl allogene als auch xenogene, d.h. aus dem Tier stammende Materialien verwendet werden können, da diese in der Regel nur zu schwachen oder keinen Immunreaktionen führen, weil die zellulären Bestandteile entfernt und die extrazellulären Matrixproteine durch die in Kontakt stehenden biologischen Gewebe umgebaut werden.

Als nicht-biologische Matrices kommen bspw. Polyanhydride, Polyphosphazine, Polyaminosäuren, Polyaktidderivate und Polyglykolderivate in Frage, die biodegradierbar oder nicht-biodegradierbar sein können.

Erfindungsgemäß kommen als physikalische Stimuli, die auf das biologische Material einwirken, solche in Frage, die ausgewählt sind aus der Gruppe bestehend aus: Zugkräfte, Druckkräfte, Scherkräfte, Zentrifugalkräfte, Zentripedalkräfte, Gravitationskräfte, Mikrogravitationskräfte. Mittels solcher Stimuli kann biologisches Material mit gewünschten Eigenschaften, wie z.B. Knochen- und Knorpelstrukturen, Herzmuskelgewebe, glattes Muskelgewebe und Skelettmuskelgewebe, gezielt generiert werden. So ist in Fachkreisen beschrieben worden, dass biologische Zellen bzw. Gewebe oder Organe durch mechanische Stimulierung gezielt verändert bzw. differenziert werden können; vgl. M. Benjamin und B. Hillen (2003), Mechanical Influences on Cells, Tissues and Organs - ,Mechanical Morphogenesis', Eur. J. Morphol. 41, Seiten 3-7. Die Wahl der jeweils gewählten Kräfte sowie die Stärke, Zeitdauer und das Muster des Stimulus hängt von den gewünschten zu generierenden biologischen Materialien ab und ist in das Belieben des Fachmannes gestellt.

Alternativ bzw. zusätzlich können als physikalische Stimuli elektrischer Strom, ein Magnetfeld, Schwerelosigkeit, Sauerstoffpartialdruck, Schallwellen, Kapillarkräfte bzw. Adhäsionskräfte bereitgestellt werden. Auch für diese Stimuli ist bekannt, dass diese biologisches Gewebe in ihrer Differenzierung gezielt steuern können. So ist bspw. bekannt, dass Muskelgewebe durch Stimulation mit bestimmten elektrischen Impulsen in seinen kontraktilen Eigenschaften kontrolliert verändert werden kann.

Die erfindungsgemäße Vorrichtung weist als Einrichtung zur Erzeugung von physikalischen Stimuli vorzugsweise einen pneumatischen, hydraulischen oder mechanischen Antrieb auf, der das biologische Material in Bewegung versetzt.

Mit dieser Maßnahme wird auf vorteilhafte Art und Weise ein physikalischer Stimulus auf das biologische Gewebe übertragen. Der pneumatische oder hydraulische Antrieb kann dabei in oder außerhalb der Kammer angeordnet sein und mit einem Zellträger in Verbindung stehen, auf dem das biologische Material angeordnet ist. Durch Inbewegungsetzen des Trägers wird der Bewegungsimpuls auf das biologische Material übertragen. Die Bewegungsform und Frequenz ist ins Belieben des Fachmanns gestellt und umfasst Translations-, Rotations-, Schwingungs-, Rüttelbewegungen u.Ä.

Bei der erfindungsgemäßen Vorrichtung ist es bevorzugt, wenn die Einrichtung zur Erzeugung von physikalischen Stimuli dadurch realisiert wird, dass die Kammer ein Kissen ist, das durch Beaufschlagung von Druck in seiner Form veränderbar ist.

Die Druckbeaufschlagung kann dabei bspw. über die Einbringung eines gasförmigen, flüssigen oder dampfförmigen Mediums, bspw. des Kulturmediums selbst, in das Kissen erfolgen. In einem solchen Kissen kann auf vorteilhafte Art und Weise sowohl die Bedampfung als auch die physikalische Stimulierung des biologischen Gewebes erfolgen. Das sich vorzugsweise an der Innenwandung des Kissens oder auf einer weiteren im Inneren des Kissens befindlichen Membran angeordnete biologische Material wird durch die Formveränderung des Kissens der entsprechend physikalisch stimuliert. Der Druck innerhalb des Kissens kann beliebig variiert werden, so dass sowohl Über- als auch Unterdrucksysteme bereitgestellt werden können. Durch gezielte Gestaltung des Kissens kann auch die Form der auf das biologische Material übertragenen Bewegung gesteuert werden. Ferner kann auf das Kissen auch eine Kraft ausgeübt werden, indem flüssige, gelartige oder gasförmige Substanzen in eine angrenzende Kammer gebracht werden und diese mit Überdruck oder Unterdruck so beaufschlagt werden, dass Sie einen Stimulus auf das Kissen ausüben.

Im Boden des Kissens selbst ist das Ultraschallwellen transmittierende Element eingesetzt, das mit dem Ultraschallwellen transmittierenden Medium in Kontakt steht. Der untere Teil bzw. der Boden des Kissens kann ferner vollständig aus dem Ultraschallwellen transmittierenden Element bestehen.

Bei einer alternativen Variante der erfindungsgemäßen Vorrichtung ist die Einrichtung zur Erzeugung von physikalischen Stimuli durch einen Rotationsantrieb realisiert, der das biologische Gewebe in Rotation versetzt.

Durch diese Maßnahme wird auf vorteilhafte Art und Weise das biologische Material Zentrifugal- oder Zentripedalkräften ausgesetzt, die das Expressionsmuster der biologischen Zellen und damit deren Differenzierung und/oder Proliferationsrate beeinflussen können. Das biologische Gewebe kann bei dieser Variante im Inneren der Kammer in einer Zentrifugentrommel angeordnet sein, die über einen Antriebsmotor mit beliebiger Geschwindigkeit in Rotation versetzt werden kann.

Alternativ kann in der erfindungsgemäßen Vorrichtung die Einrichtung zur Erzeugung von physikalischen Stimuli ein Mittel aufweisen, das in Kontakt mit dem biologischen Material ist und auf dieses mechanischen Druck ausübt, oder eine Gas- oder Flüssigkeitsdüse aufweisen, über die auf das biologische Material Gas- oder Flüssigkeitsdruck ausgeübt wird.

Mit diesen Varianten kann das biologische Material auf andere Art und Weise physikalisch stimuliert und damit in dessen Entwicklung andersartig beeinflusst werden.

Weitere Alternativen bestehen darin, dass die Einrichtung zur Erzeugung von physikalischen Stimuli durch einen Spannungsgenerator oder einen Magneten realisiert wird, der dem biologischen Material elektrische Impulse zuführt bzw. das biologische Material einem Magnetfeld aussetzt.

Ferner kann in der erfindungsgemäßen Vorrichtung die Einrichtung zur Erzeugung von physikalischen Stimuli kontraktile biologische Zellen aufweisen, die mit dem biologischen Material in Kontakt stehen.

Diese Variante hat den Vorteil, dass keine weiteren mechanisch-konstruktiven Vorkehrungen an der Vorrichtung getroffen werden müssen, die Stimulierungseinrichtung vielmehr durch "biologische Einheiten" realisiert wird. Die kontraktilen Zellen können in der Kammer vielmehr gleichermaßen mit Kulturmedium versorgt und kultiviert werden, wie das biologische Material.

Die erfindungsgemäße Vorrichtung kann einen Temperaturregler zur Temperierung des Kulturmediums aufweisen.

Mit dieser Maßnahme lässt sich auf vorteilhafte Art und Weise das Kulturmedium unabhängig von der Raumtemperatur auf die gewünschte Temperatur bringen, so dass optimalen Bedingungen für die Generierung von Gewebekonstrukten eingestellt werden können.

Die erfindungsgemäße Vorrichtung kann eine Gaszufuhr zur Begasung des biologischen Gewebes aufweisen.

Durch diese Maßnahme wird gewährleistet, dass das biologische Material hinreichend mit den für die Generierung von Gewebekonstrukten erforderlichen Gasen, wie bspw. Sauerstoff oder Kohlendioxid, versorgt wird. Dabei kann die erfindungsgemäße Vorrichtung Messsonde zur Erfassung physiologischer Parameter aufweisen, die bspw. der permanenten Überprüfung der Gasmengen und des Gasdrucks, der Glucose-, Laktatkonzentrationen etc. dienen. Eine wahlweise vorgesehene Regeleinrichtung dient der Regulierung des Gasdrucks und der Gasmenge in der Kammer. Derartige Messsonden und Regeleinrichtungen sind dem Fachmann bekannt.

Das erfindungsgemäße Verfahren lässt sich auf vorteilhafte Weise mit der erfindungsgemäßen Vorrichtung durchführen.

Ein weiterer von den Erfindern entwickelter Gegenstand betrifft eine Vorrichtung zur Generierung von biologischem Material, die Folgendes aufweist, nämlich: eine Kammer, die biologisches Material aufnehmen kann, und eine Einrichtung zur Einbringung von Tröpfchen eines Kulturmediums in der Kammer, wobei die Vorrichtung ferner eine Einrichtung zur Erzeugung von auf das biologische Material einwirkende physiologischen oder/und physikalischen Stimuli aufweist.

Mit dieser Vorrichtung kann auf besonders vorteilhafte Art und Weise das erfindungsgemäße Verfahren durchgeführt werden. Eine solche Vorrichtung steht im Stand der Technik bislang nicht zur Verfügung. So sehen bspw. die Vorrichtungen aus den eingangs erwähnten EP 0 234 868 B1, JP 59 045 879 A und US 4,332,105 keine Einrichtung zur Erzeugung von auf das biologische Material einwirkende physiologischen oder/und physikalischen Stimuli vor und ermöglichen insbesondere nicht die erfindungsgemäße Generierung von biologischem Material. Die bekannten Vorrichtungen sind vielmehr zur reinen Kultivierung von Pflanzenzellen oder tierischen Monolayerkulturen eingerichtet.

Bei der weiteren Vorrichtung wird die Einrichtung zur Erzeugung von physiologischen Stimuli vorzugsweise durch eine Vorrichtung zur Zufuhr von Wachstumsfaktoren in das Kulturmedium bzw. alternativ durch eine mit dem biologischen Material in Kontakt stehende biologische Matrix oder/und eine nicht-biologische Matrix realisiert.

Bei der weiteren Vorrichtung kann die Einrichtung zur Erzeugung von physikalischen Stimuli vorzugsweise einen pneumatischen oder hydraulischen oder mechanischen Antrieb aufweisen, der das biologische Material in Bewegung versetzt.

Bei der weiteren Vorrichtung kann die Einrichtung zur Erzeugung von physikalischen Stimuli alternativ oder zusätzlich ein Kissen aufweisen, in dem das biologische Material generiert wird, das durch Beaufschlagung von Druck in seiner Form veränderbar ist, wobei das Kissen vorzugsweise so ausgestaltet ist, dass die Tröpfchen des Kulturmediums in den Innenraum des Kissens eingebracht werden können.

Bei der weiteren Vorrichtung kann die Einrichtung zur Erzeugung von physikalischen Stimuli ferner einen Rotationsantrieb aufweisen, der das biologische Material in Rotation versetzt, oder ein Mittel aufweisen, das in Kontakt mit dem biologischen Material ist und auf dieses mechanischen Druck ausübt.

Ferner kann die Einrichtung zur Erzeugung von physikalischen Stimuli der weiteren Vorrichtung eine Gas- oder Flüssigkeitsdüse aufweisen, über die auf das biologische Material Gas- oder Flüssigkeitsdruck ausgeübt wird.

Bei der weiteren Vorrichtung kann die Einrichtung zur Erzeugung von physikalischen Stimuli alternativ oder zusätzlich einen Spannungsgenerator aufweisen, der dem biologischen Material elektrische Impulse zuführt, oder einen Magneten aufweisen, der das biologische Material einem Magnetfeld aussetzt.

Bei der weiteren Vorrichtung kann die Einrichtung zur Erzeugung von physikalischen Stimuli außerdem oder zusätzlich kontraktile biologische Zellen aufweisen, die mit dem biologischen Material in Kontakt stehen.

In einer Weiterbildung der weiteren Vorrichtung weist die Einrichtung zur Erzeugung von Tröpfchen einen Ultraschallzerstäuber auf.

Diese Maßnahme hat sich als für die Erzeugung von Flüssigkeitströpfchen besonders geeignet herausgestellt. Dazu kann ein Ultraschallgenerator vorgesehen sein, der im Inneren der Kammer direkt in das Kulturmedium eingebracht wird und durch Anregung eines piezokeramischen Elements mit Frequenzen im Ultraschallbereich dieses so in Schwingung versetzt, dass die dadurch verursachten Wellenbewegungen im Medium zu einer Vernebelung des Mediums im Inneren der Kammer führt. Alternativ kann der Ultraschallgenerator eine Düse bzw. einen Schwingkopf aufweisen. Diesem Schwingkopf kann Kulturmedium so zugeführt werden, dass dieses durch die Vibration des Schwingkopfs zerstäubt und in der Kammer vernebelt wird. In einer weiteren Ausführungsvariante kann der Ultraschallzerstäuber das Medium außerhalb der Kammer in einer Nebenkammer zerstäuben. Der generierte Nebel wird in dieser Ausführungsvariante erst nach der Zerstäubung der eigentlichen Kulturkammer zugeführt. Ultraschallzerstäuber sind im Stand der Technik allgemein bekannt. Beispielhaft wird auf die bereits genannten EP 0 234 868 B1, JP 59 045 879 A und US 4,332,105 verwiesen.

An der weiteren Vorrichtung kann ein Temperaturregler zur Temperierung des Kulturmediums bzw. eine Gaszufuhr zur Begasung des biologischen Materials vorgesehen sein.

Die Erfinder haben außerdem ein weiteres Verfahren zum Kultivieren und Generieren von biologischem Material entwickelt, das folgende Schritte aufweist: (a) Bereitstellen von biologischem Material; (b) Einbringen des biologischen Materials in eine Kammer; (c) Einbringen von Tröpfchen eines Kulturmediums in die Kammer derart, dass das biologische Material mit den Tröpfchen in Kontakt kommt, und (d) physiologische oder/und physikalische Stimulierung des biologischen Materials.

Die physiologische Stimulierung kann in dem weiteren Verfahren durch In-Kontakt-Bringen des biologischen Materials mit Wachstumsfaktoren realisiert werden, die vorzugsweise in dem Kulturmedium bereitgestellt werden.

Die physiologische Stimulierung kann in dem weiteren Verfahren ferner durch In-Kontakt-Bringen des biologischen Materials mit einer biologischen Matrix oder/und einer nicht-biologischen Matrix realisiert werden.

Die physikalischen Stimuli, die in dem weiteren Verfahren auf das biologische Material einwirken, sind vorzugsweise ausgewählt aus der Gruppe bestehend aus: Zugkräfte, Druckkräfte, Scherkräfte, Zentrifugalkräfte, Zentripedalkräfte, Gravitationskräfte, Mikrogravitationskräfte, Elektrischer Strom, Magnetfeld, Schwerelosigkeit, Sauerstoffpartialdruck, Schallwellen, Kapillarkräfte, Adhäsionskräfte.

Hinsichtlich der Merkmale und Vorteile der weiteren von den Erfindern entwickelten Vorrichtung bzw. des weiteren Verfahrens gelten die oben im Zusammenhang mit der erfindungsgemäßen Vorrichtung und dem erfindungsgemäßen Verfahren gemachten Angaben entsprechend.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun anhand von Ausführungsbeispielen näher erläutert, die rein illustrativen Charakter haben und die Tragweite der Erfindung nicht einschränken. Dabei wird Bezug genommen auf die beigefügten Figuren, in denen zu sehen ist:
- Fig. 1: eine schematische Übersicht (A) über die weitere Vorrichtung mit einer ersten Ausführungsform des Ultraschallgenerators (B);
- Fig. 2: eine schematische Übersicht (A) über die weitere Vorrichtung mit einer zweiten Ausführungsform des Ultraschallgenerators (B);
- Fig. 3: eine schematische Übersicht über eine Variante der weiteren Vorrichtung bei der der Nebel in einer separaten Kammer produziert wird und der Kulturkammer zugeführt wird;
- Fig. 4: schematische Übersichten über die weitere Vorrichtung, die in einen Zellkulturschrank eingebracht ist;
- Fig. 5: verschiedene Varianten von Zellträgern;
- Fig. 6: Beispiele für verschiedene biologische Materialien;
- Fig. 7: eine schematische Übersicht über die weitere Vorrichtung mit einer Matrixkomponente und Wachstumsfaktoren als physiologische Stimuli;
- Fig. 8: eine schematische Übersicht über die weitere Vorrichtung mit einer ersten Ausführungsform einer Einrichtung zur physikalischen Stimulierung des biologischen Materials;
- Fig. 9: verschiedene weitere Ausführungsformen der Einrichtung zur physikalischen Stimulierung des biologischen Materials als Bestandteil der weiteren Vorrichtung;
- Fig. 10: verschiedene Ausführungsformen der Einrichtung zur physikalischen Stimulierung des biologischen Materials als Bestandteil der erfindungsgemäßen Vorrichtung;
- Fig. 11: eine schematische Übersicht über Ausführungsformen der erfindungsgemäßen Vorrichtung als Zweikammersystem;
- Fig. 12: eine weitere schematische Übersicht über Ausführungsformen der erfindungsgemäßen Vorrichtung, in denen die Kammer durch ein Kissen realisiert ist und der Ultraschallbrennpunkt in seiner Position veränderbar ist;
- Fig. 13: eine schematische Darstellung einer Ausführungsform eines Kissens zur physikalischen Stimulierung und Mediumsbedampfung des biologischen Materials;
- Fig. 14, 16 und 17: experimentelle Nachweise über die physiologische Funktionsfähigkeit und Vitalität von verschiedenen biologischen Materialien durch das erfindungsgemäße Verfahren, und
- Fig. 15: die Eindämmung von bakterieller Verunreinigung durch das, erfindungsgemäße Verfahren.

### Ausführungsbeispiel 1

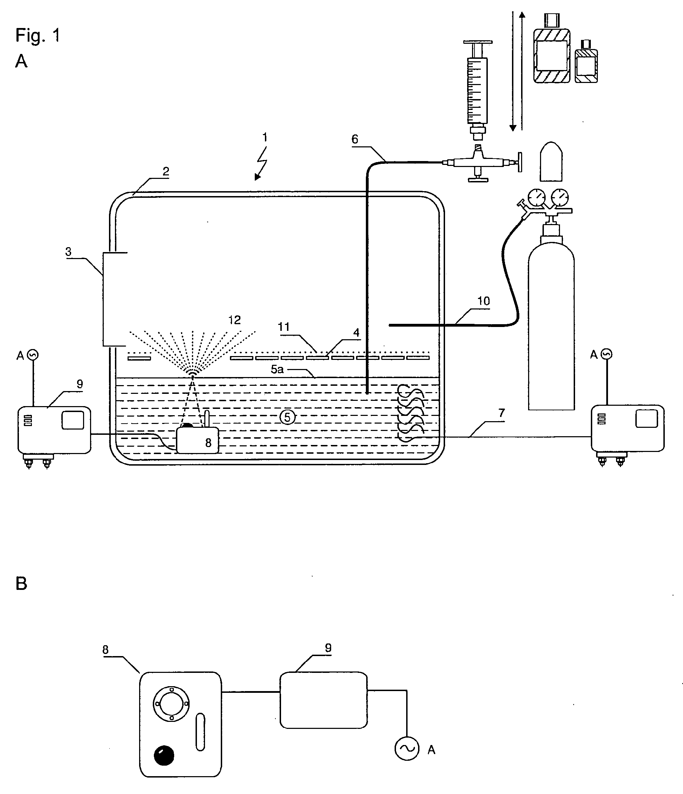

In Fig. 1A ist eine erste Ausführungsform einer Gewebegenerierungsvorrichtung 1 dargestellt. Diese weist eine Kammer 2 in Form eines üblichen Kulturgefäßes auf. Ein seitlich angebrachter Öffnungsflügel 3 ermöglicht einen Zugriff in das Innere der Kammer 2. Im Inneren der Kammer 2 befindet sich ein Träger 4. Über dem Boden der Kammer 2 befindet sich Kulturmedium 5, das über eine außerhalb der Kammer 2 angeordnete Medienzu- und -abfuhr 6, die aus einem Vorratsbehälter für das Kulturmedium 5 und einem Schlauch besteht, in das innere der Kammer 2 regulierbar eingebracht werden kann. Die Temperatur des Kulturmediums 5 ist über eine Heizspirale einer Temperaturregeleinheit 7 einstellbar. Die Oberfläche des Kulturmediums 5 bildet im Inneren der Kammer 2 ein Flüssigkeitskante 5a.

Auf dem Boden der Kammer 2 befindet sich innerhalb des Kulturmediums 5 ein Ultraschallgenerator 8, der mit einem Spannungs- und Frequenzregler 9 verbunden ist. Der Ultraschallgenerator 8 und der Spannungs- und Frequenzregler 9 sind in Teilabbildung Fig. 1B im Detail dargestellt. Der Ultraschallgenerator (8) weist einen Piezokeramikschwinger auf. Die Piezokeramik wird durch eine hochfrequente Wechselspannung in Schwingungen versetzt. Es bilden sich Ultraschallwellen aus, die ihre maximale Stärke auf einer bestimmten Flüssigkeitshöhe haben und die das Entstehen eines Ultraschallrüssels bewirken. Aus diesem Ultraschallrüssel lösen sich kleine Flüssigkeitströpfchen - das Aerosol bzw. ein Nebel 12 - das bzw. der dann für die gewünschte Anwendungen genutzt werden kann. Die Kammer 2 wird deshalb auch als Nebelkammer bezeichnet. Vorzugsweise ist ein bestimmtes Flüssigkeitsniveau, bspw. von 55 mm, mit einer Genauigkeit von bspw. ± 5 mm, konstant zu halten. Dies kann z.B. durch einen Schwimmerschalter oder nach dem Überlaufprinzip geschehen. Die Menge des erzeugten Aerosols bzw. Nebels 12 kann über eine externe Steuerspannung oder ein Potentiometer verändert werden.

Über eine Gaszufuhr 10 lassen sich in die Kammer 2 beliebige Gase, wie bspw. Sauerstoff und Kohlendioxid einbringen.

Auf dem Träger 4 angeordnet befindet sich biologisches Material 11, bspw. biologische Zellen. Bei Inbetriebnahme des Ultraschallgenerators 8 bildet sich, wie vorstehend erläutert, in der Kammer 2 der Nebel 12, bestehend aus feinen Tröpfchen des Kulturmediums 5. In dem Kulturmedium 5 können sich als physiologische Stimuli Wachstumsfaktoren befinden, die über die Medienzu- und -abfuhr 6 in gewünschter Konzentration zumischbar sind. Über den Nebel 12 wird das Material 11 optimal mit Kulturmedium 5 und ggf. den darin gelösten Wachstumsfaktoren versorgt.

Die Dauer der Bestäubung bzw. Stimulierung des Materials 11 kann nach Belieben variiert werden und hängt insbesondere von de Art des biologischen Materials 11 bzw. der zu generierenden Gewebekonstrukte ab.

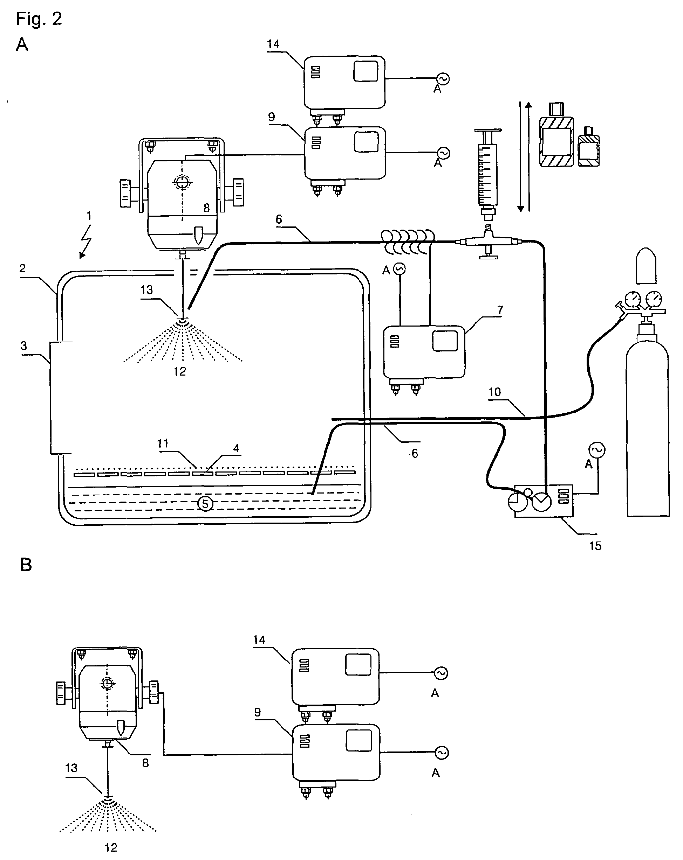

In Fig. 2A ist unter Verwendung der entsprechenden Bezugszeichen eine alternative Ausführungsform der Gewebegenerierungsvorrichtung 1 dargestellt, bei der Ultraschallgenerator 8 außerhalb der Kammer 2 angeordnet ist und zur Generierung des Nebels 12 aus Kulturmedium 5 erstreckt sich ausgehend von dem Ultraschallgenerator 8 durch eine Öffnung in der Decke der Kammer 2 ein Schwingkopf 13 in das Kammerinnere. Die Medienzu- und -abfuhr 6 leitet das Kulturmedium 5 direkt auf den Schwingkopf 13.

Diese Variante des Ultraschallgenerators ist in Teilabbildung Fig. 2B im Detail dargestellt. Ein piezokeramisches Element wandelt elektrische Wellen in mechanische Schwingungen um. Die drucklos auf den Schwingkörper aufgebrachte Flüssigkeit bzw. das Kulturmedium 5 bildet Kapillarwellen, aus denen feinste Tropfen abgenebelt (Nebel 12) werden.

Sowohl die in Fig. 1A als auch in Fig. 1B dargestellte Varianten weisen verschiedene Vorteile auf: Sehr geringer Energieaufwand durch drucklose Zuführung des Mediums 5, geringer elektrischer Leistungsbedarf, gleichmäßige Zerstäubung veränderlicher Volumenströme von Maximum bis annähernd Null, nahezu konstantes Tropfenspektrum über den gesamten Volumenstrombereich, hohe Betriebssicherheit, da praktisch verstopfungsfrei, geringe Eigengeschwindigkeit der Tropfen und dadurch gute Beeinflussbarkeit des Strahls, betriebssicherer Oszillator.

Bei der in Fig. 2 dargestellten Ausführungsform der Gewebegenerierungsvorrichtung 1 weist die Medienzu- und Abfuhr 6 ferner eine Peristaltikpumpe 15 auf, über die das Kulturmedium 5 aus der Kammer 2 auch geregelt abführen kann, so dass ein Durchlaufsystem bereitgestellt wird.

Die Heizspirale der Temperaturregeleinheit 7 setzt bei dieser Variante außerhalb der Kammer 2 an dem Schlauch der Medienzu- und Abfuhr 6 an, die das Kulturmedium 5 der Kammer 2 zuführt.

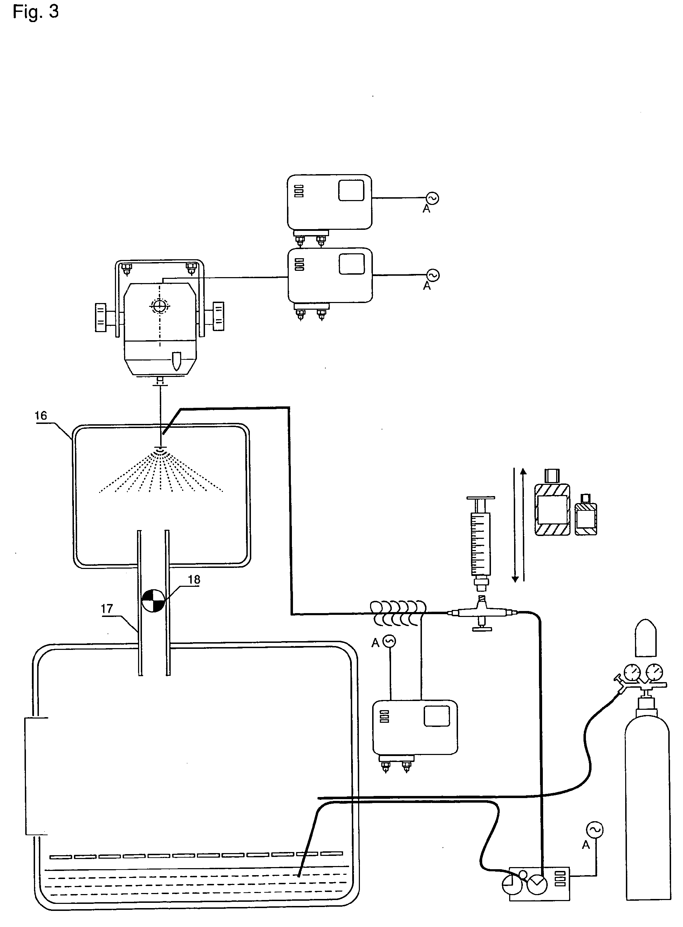

Fig. 3 stellt eine weitere Ausführungsform der Gewebegenerierungsvorrichtung 1 dar, bei der der Nebel 12 außerhalb der Kammer 2 in einer separaten Kammer 16 erzeugt und anschließend über eine Verbindungseinheit 17, in der ein Ventilator 18 angeordnet ist, in die Kammer 2 eingebracht wird. Diese konstruktive Alternative hat den Vorteil, dass ein Ultraschallgenerator 8 für mehrere Kammern 2 verwendet werden kann. Ferner eine vereinfachte Wartung des Ultraschallgenerators 8 möglich; der Ultraschallgenerator 8 kann außerhalb der Kammer 2 aufgestellt werden, so dass herkömmliche Inkubatoren als Kammer 2 verwendet werden können und die Vernebelung nur als Zusatzsystem dient das mit vorhandenem kooperiert. Das Kontaminationsrisiko ist bei dieser Variante weiter vermindert.

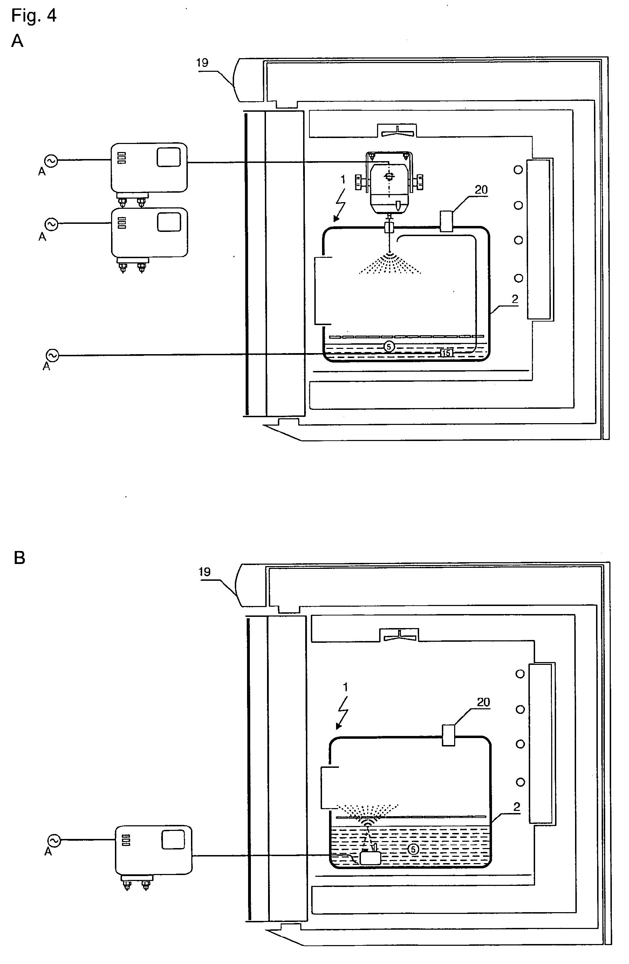

In Fig. 4A und Fig. 4B sind weitere Varianten der Gewebegenerierungsvorrichtung 1 dargestellt, bei der letztere in einen Zellkulturschrank 19 eingebracht ist, über die die Temperatur- und Gasversorgung kontrolliert wird. Auf eine separate Temperaturregeleinheit 7 und ggf. Gaszufuhr 10 kann bei dieser Variante verzichtet werden.

In der Decke der Kammer 2 ist bei dieser Variante ein Filter 20 vorgesehen, der die Funktion hat, die Gasdiffusion zum Erhalt der in dem Zellkulturschrank 19 erzeugten Atmosphäre bis in die eigentliche Kammer 2 zu ermöglichen. Die Kammer 2 muss so nicht separat konditioniert werden. Der Filter 20 sorgt ferner für eine sterile Abdichtung der Kammer 2.

Teilabbildung Fig. 4A zeigt die Variante der Gewebegenerierungsvorrichtung 1, bei der ein Schwingkopf 13 als Bestandteil des Ultraschallgenerators 8 vorgesehen ist, wohingegen Teilabbildung Fig. 4B den sich im Kulturmedium 5 befindlichen Ultraschallgenerator 8 aufweist. Teilabbildung Fig. 4A zeigt ferner eine Variante der Medienzu- und -abfuhr 6, bei der sich die Peristaltikpumpe 15 im Inneren der Kammer 2 befindet und das Kulturmedium 5 direkt im Kurzschluss vom Boden der Kammer 2 zum Schwingkopf 13 geführt wird.

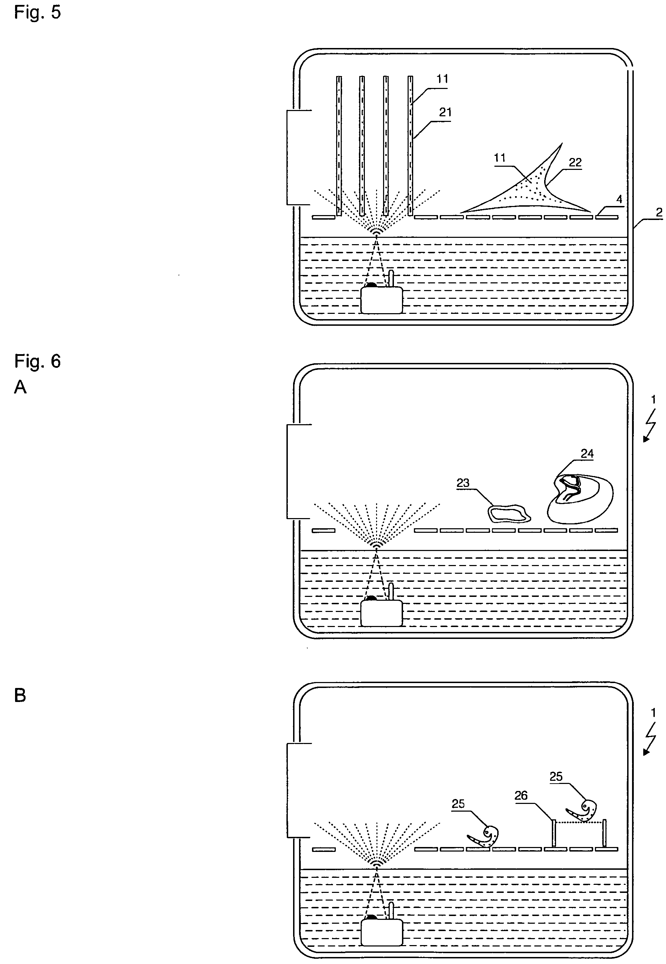

In Fig. 5 sind neben dem horizontal angeordneten Träger Varianten weiterer Träger dargestellt, nämlich ein im Inneren der Kammer 2 vertikal angeordneter Zellträger 21 und ein räumlich gekrümmter Zellträger 22, auf denen das biologische Material 11 angeordnet ist.

In Fig. 6A sind als biologisches Material Gewebe bzw. Gewebekonstrukte 23 sowie Organe 24 dargestellt, die mit der Gewebegenerierungsvorrichtung 1 kultiviert bzw. generiert werden können. Teilabbildung Fig. 6B zeigt Embryonen 25, die entweder direkt auf den Träger 4 oder auf eine Trägermembran 26 aufgebracht und mit der Gewebegenerierungsvorrichtung 1 kultiviert bzw. generiert werden können.

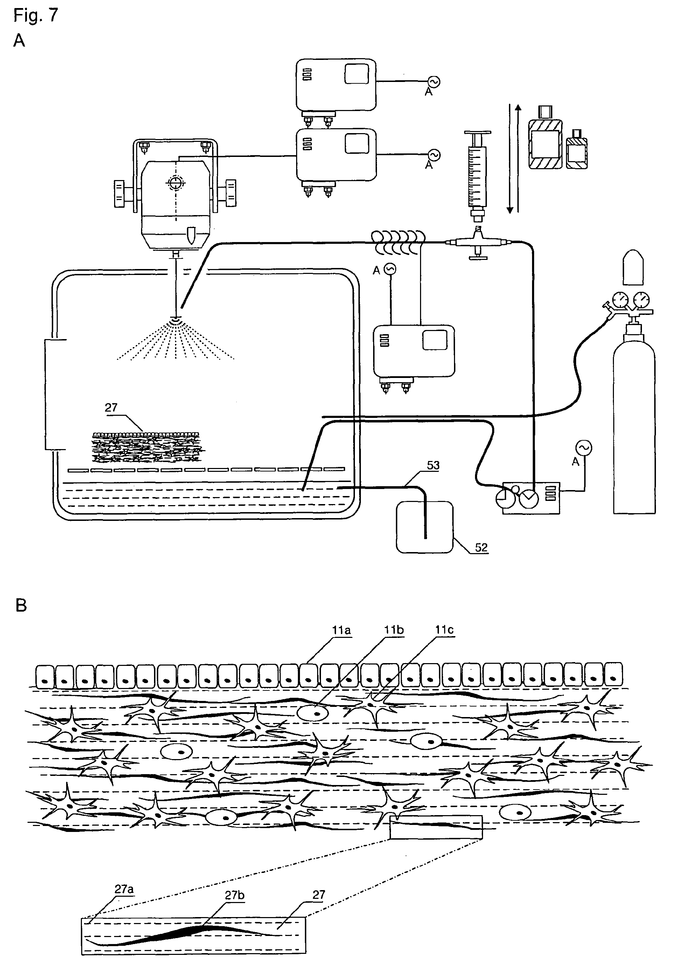

Fig. 7A zeigt die Gewebegenerierungsvorrichtung 1, die eine Matrixkomponente 27 aufweist, die das biologische Material 11 physiologisch stimuliert. Wie in Teilabbildung Fig. 7B näher dargestellt, steht die Matrixkomponente 27, bei der es sich um eine biologische oder nicht-biologische Matrix 27a handeln kann, die zellproliferations- und zelldifferenzierungsfördernde Wachstumsfaktoren 27b aufweisen kann, in direktem Kontakt mit dem biologischen Material 11 und vermittelt dadurch die physiologischen Stimuli. Die Matrixkomponente 27 kann mit verschiedenen Zelltypen 11a, 11b, 11c in Kontakt stehen.

Alternativ oder zusätzlich werden die Wachstumsfaktoren über eine separate Einrichtung bzw. Vorrichtung in die Kammer 2 eingebracht. So werden die Wachstumsfaktoren in einem Behälter 52 in gelöster Form bereitgestellt und über eine Zufuhrleitung 53 in das Innere der Kammer 2 bzw. in das Kulturmedium 5 eingebracht. Dieses wird dann über einen Kreislauf zum Ultraschallgenerator 8 geführt, der das Medium mit den Wachstumsfaktoren zerstäubt. Die Wachstumsfaktoren kommen dann in Kontakt mit dem biologischen Material 11.

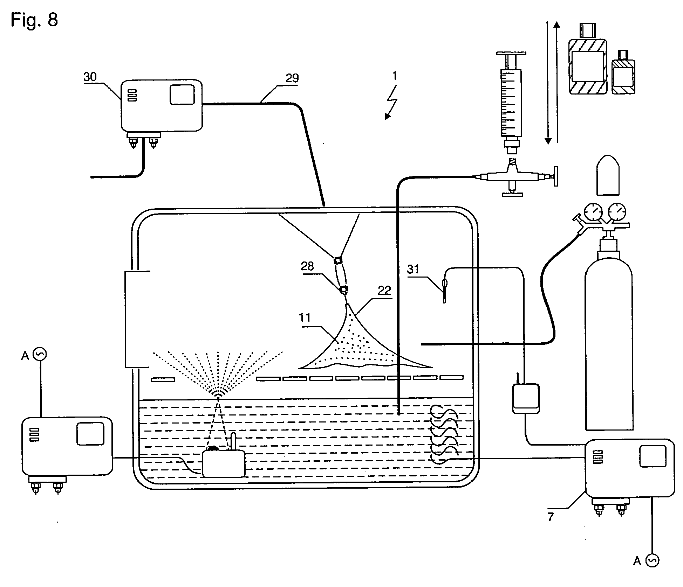

Fig. 8 zeigt eine weitere Variante der Gewebegenerierungsvorrichtung 1, die einen pneumatischen Antrieb 28 aufweist, der über eine Druckluftleitung 29 mit einem Druckluftgenerator 30 verbunden ist. Der pneumatische Antrieb 28 ist im Inneren der Kammer 2 mit dem räumlich gekrümmten Zellträger 22 verbunden. Bei Beaufschlagung des pneumatischen Antriebs 28 mit Druckluft über den Druckluftgenerator 30 bzw. die Druckluftleitung 29 führt der pneumatische Antrieb eine Translationsbewegung aus, die zu einer Bewegung des räumlich gekrümmten Zellträgers 22 und dadurch zu einer physikalischen Stimulierung des darauf angeordneten biologischen Materials 11 führt.

In Fig. 8 ist ferner ein Temperaturmessfühler 31 angeordnet, der mit der Temperaturregeleinheit 7 verbunden ist und dieser Information bereitstellt, so dass bei einem Absinken der Temperatur unter einen Soll-Wert die Heizspirale der Temperaturregeleinheit 7 das Kulturmedium 5 wieder auf die gewünschte Temperatur bringt.

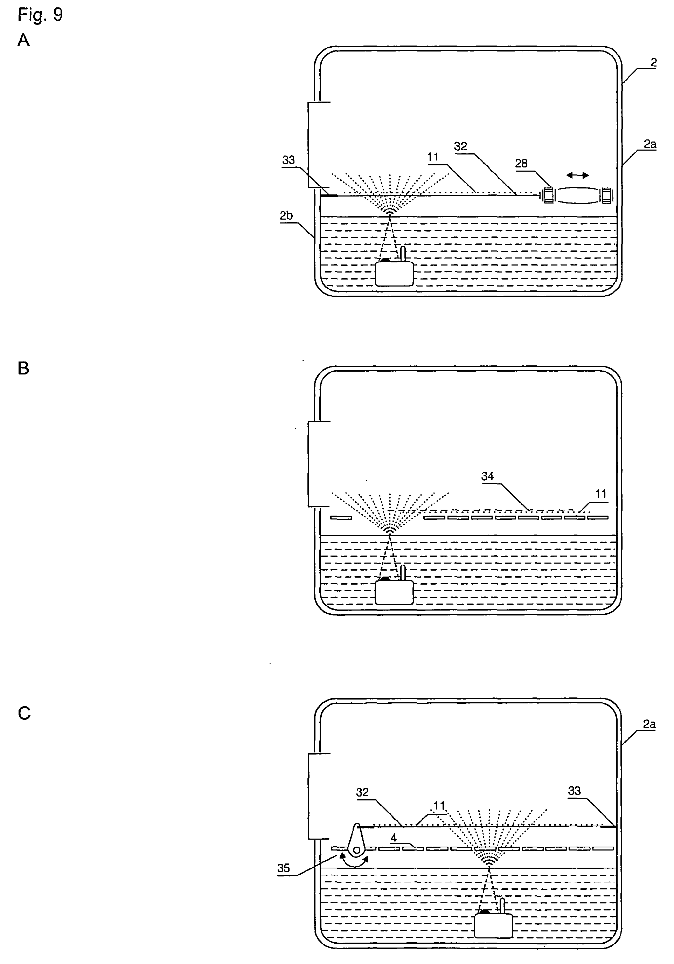

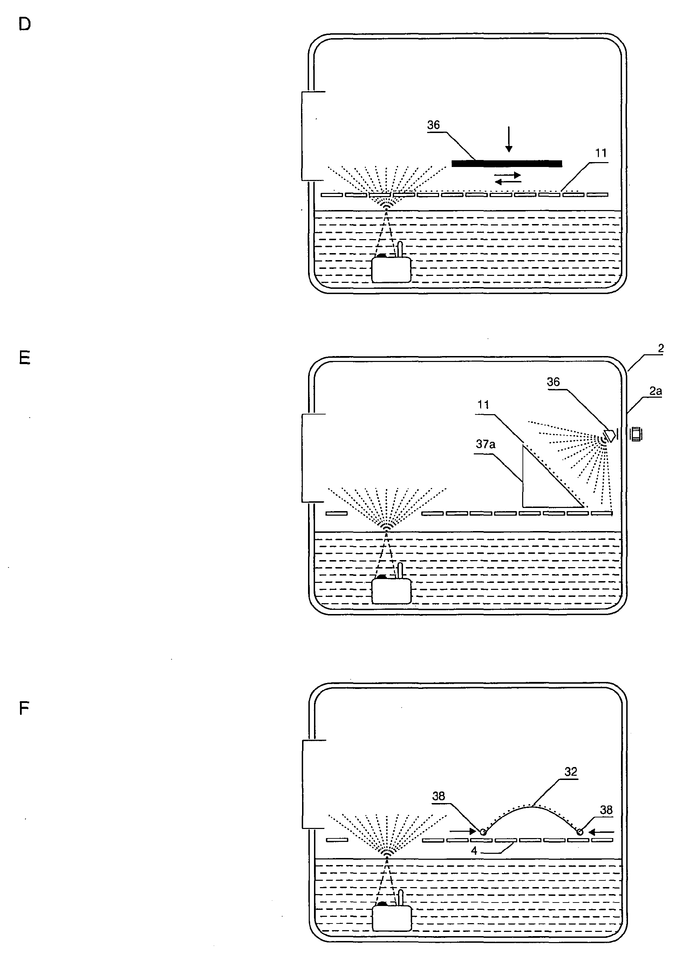

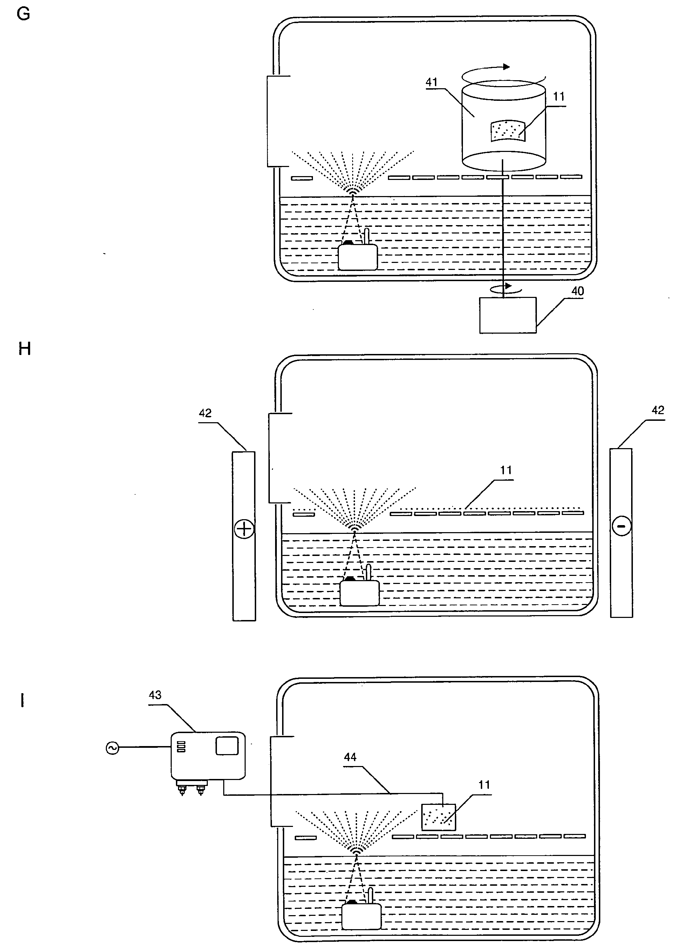

In Fig. 9 sind weitere Varianten der Einrichtung zur Erzeugung von physikalischen Stimuli dargestellt. Teilabbildung Fig. 9A zeigt den pneumatischen Antrieb 28, bei dem es sich auch um einen hydraulischen Antrieb handeln kann, der auf einer Seite mit der Wandung 2a der Kammer 2 befestigt ist. Die andere Seite des pneumatischen Antriebs 28 ist mit einem flexiblen Träger 32 verbunden, der mit seinem anderen Ende an der Wandung 2b der Kammer 2 verbunden ist, die der Wandung 2a gegenüberliegt. Auf dem flexiblen Träger 32 ist das biologische Material 11 angeordnet. Wenn der pneumatische Antrieb 28 in Betrieb genommen wird, führt dieser eine translatorische Bewegung aus, die durch den Doppelpfeil angedeutet ist. Diese wird auf den flexiblen Träger 32 übertragen, der sich gleichermaßen in Bewegung setzt, wodurch das darauf angeordnete biologische Material 11 physikalisch stimuliert wird. Wie aus dieser Teilabbildung Fig. 9A ersichtlich, kann bei der Gewebegenerierungsvorrichtung 1 das biologische Material 11 gleichzeitig physikalisch und physiologisch stimuliert werden, nämlich indem bspw. in dieser Ausführungsform dem Kulturmedium 5 Wachstumsfaktoren zugesetzt werden.

Teilabbildung Fig. 9B zeigt eine Variante der physikalischen Stimulierung des biologischen Materials 11. Bei dieser Variante sind kontraktile Zellen 34 vorgesehen, die in direktem Kontakt mit dem auf dem Träger 4 angeordneten biologischen Material 11 stehen. Durch die Kontraktion der kontraktilen Zellen 34 wird die Bewegung als physikalischer Stimulus auf das Material 11 übertragen.

Bei dieser Variante können über die kontraktilen Zellen 34 gleichzeitig physiologische Stimuli bereitgestellt werden, nämlich über die extrazelluläre Matrix der kontraktilen Zellen 34, die dann der Matrixkomponente 27 entspricht.

In der Teilabbildung Fig. 9C ist als Einrichtung zur Erzeugung von physikalischen Stimuli ein mechanischer Antrieb 35 dargestellt. Dieser ist an seiner unteren Seite gelenkig mit dem Träger 4 verbunden und mit der dieser gegenüberliegenden Seite mit einem Ende des flexiblen Trägers 32, auf dem das biologische Material 11 angeordnet ist. Der flexible Träger 32 ist mit seinem anderen Ende an der Wandung 2a der Kammer 2 verbunden. Bei Inbetriebnahme des mechanischen Antriebs 35 führt dieser eine Schwenkbewegung aus, die durch den Doppelpfeil angedeutet ist. Dadurch wird der flexible Träger 32 in entsprechende Bewegung versetzt, die als physikalischer Stimulus auf das biologische Material 11 übertragen wird.

In Teilabbildung Fig. 9D ist ein weiterer mechanischer Antrieb 36 dargestellt, der mit dem Material 11 in Kontakt steht und auf dieses mechanischen Druck oder mechanischen Scherstress ausübt, wie dies durch die Pfeile angedeutet ist.

In Teilabbildung Fig. 9E ist als Einrichtung zur Erzeugung von physikalischen Stimuli eine Gas- oder Flüssigkeitsdüse 37 dargestellt, die an der Wandung 2a der Kammer 2 befestigt ist und Flüssigkeit oder Gas auf das biologische Material 11 mit einem gewünschten Druck abgibt und dadurch bei letzterem Druck oder Scherstress als physikalische Stimuli verursacht. Bei dieser Variante sind das biologische Material 11 auf einer separaten Halterung 37a angeordnet, so dass diese in optimaler Ausrichtung zu der Flüssigkeit bzw. dem Gas ausgerichtet sind.

Teilabbildung Fig. 9F zeigt eine weitere Variante der Einrichtung zur Erzeugung von physikalischen Stimuli, bei der ein mechanischer Antrieb 38 vorgesehen ist, der derart ausgestaltet ist, dass dieser von beiden Seiten auf den flexiblen Träger 32 Druck ausübt, wie durch die beiden Pfeile angedeutet, wodurch sich der flexible Träger 32 verbiegt, d.h. eine Biegespannung induziert wird, die als physikalischer Stimulus auf das biologische Material 11 übertragen wird.

In der Teilabbildung Fig. 9G weist die Einrichtung zur Erzeugung von physikalischen Stimuli einen Rotationsantrieb 40 auf, der in dieser Ausführungsform außerhalb der Kammer 2 angeordnet ist. Über eine Achse, die ins Innere der Kammer 2 führt, wird eine Rotationsbewegung, die über dem Pfeil angedeutet ist, auf einen Behälter 41 übertragen, in dem sich das biologische Material 11 befindet. Das biologische Material 11 wird dadurch in Rotationsbewegung versetzt und der Zentrifugalkraft unterworfen.

In Teilabbildung Fig. 9H ist die Einrichtung zur Erzeugung von physikalischen Stimuli durch einen Magneten 42 realisiert, der außerhalb der Kammer 2 angeordnet ist und das biologische Material 11 einem Magnetfeld aussetzt.

In Teilabbildung Fig. 9I ist die Einrichtung zur Erzeugung von physikalischen Stimuli durch eine Spannungsgenerator 43 realisiert, der außerhalb der Kammer 2 angeordnet ist und über eine Leitung 44 dem biologischen Material 11 elektrische Impulse zuführt.

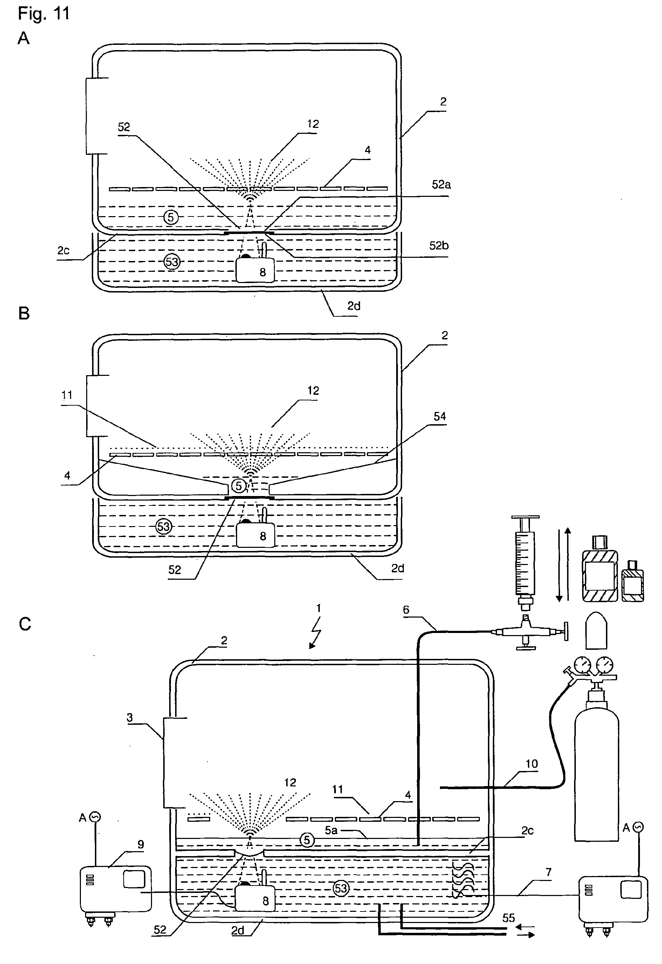

Fig. 11 zeigt schematische Darstellungen der erfindungsgemäßen Vorrichtung in Form eines Zweikammersystems. Die einzelnen Elemente entsprechen denen aus den Vorrichtungen, die in den vorherigen Figuren dargestellt sind. Die Bezugsziffern wurden, soweit möglich, beibehalten.

Die in Teilabbildung 11A dargestellte Kammer 2 enthält das Kulturmedium 5. Die Kammer 2 weist einen Boden 2c auf, in den dichtend das Ultraschallwellen transmittierende Element 52 in Form einer Platte bestehend aus einem Ultraschalltransmittierendem Material, wie einem Metall, eingesetzt ist. Das Kulturmedium 5 steht in direktem Kontakt mit einer ersten Oberfläche 52a des Elementes 52. In einer nach unten angrenzenden zweiten Kammer 2d befindet sich ein Ultraschallwellen transmittierendes Medium 53, das in direktem Kontakt mit einer zweiten Oberfläche 52b des Elementes 52 steht. Die zweite Kammer 2d kann auch als Transmissionskammer bezeichnet werden. Im Ultraschallwellen transmittierendes Medium 53 befindet sich der Ultraschallgenerator 8, der Ultraschallwellen in das Ultraschallwellen transmittierende Medium 53 einleitet. Diese werden von der zweiten Oberfläche 52b des Ultraschallwellen transmittierenden Elementes 52 aufgenommen und über die erste Oberfläche 52a des Ultraschallwellen transmittierenden Elementes 52 in das Kulturmedium 5 in der Kammer 2 weitergeleitet. In der Kammer 2 entsteht dadurch das Aerosol bzw. der Nebel 12 aus Kulturmedium 5.

In Teilabbildung Fig. 11B ist eine Ausführungsform der erfindungsgemäßen Vorrichtung dargestellt, bei der in der Kammer 2 ein trichterförmiges Reduzierstück 54 vorgesehen ist. Dieses erstreckt sich ausgehend von der Oberfläche 52a des Ultraschallwellen transmittierenden Elementes 52 trichterförmig nach oben in Richtung des sich auf dem Träger 4 befindlichen biologischen Materials 11. Die Basis des Reduzierstückes 54 ist dabei dichtend mit dem Teil des Bodens 2d der Kammer 2 verbunden, der das Ultraschallwellen transmittierende Element 52 aufweist. Die Basis des Reduzierstückes 54 kann auch direkt auf dem Ultraschallwellen transmittierenden Element 52 angeordnet sein. Durch diese Maßnahme kann die Menge des sich in der Kammer 2 befindlichen Kulturmediums reduziert werden.

In Teilabbildung Fig. 11C ist eine Übersicht der erfindungsgemäßen Vorrichtung entsprechend der Fig. 1A dargestellt, auf die verwiesen wird. Das Ultraschallwellen transmittierende Elementes 52 ist in Form einer Membran dargestellt, die konvex in Richtung des Ultraschallwellen transmittierenden Mediums 53 gewölbt ist. Die Temperatur des Kulturmediums 5 wird indirekt über die Heizspirale der Temperaturregeleinheit 7 eingestellt, die in das Ultraschallwellen transmittierende Medium eingebracht ist. Die dort erzeugte Wärme wird über den Boden 2c der Kammer 2 in das Kulturmedium 5 übertragen. Dadurch wird eine Kontamination des Kulturmediums 5 durch die Temperaturregeleinheit 7 vermieden. Unter der Bezugsziffer 55 sind Zu- und Abfuhrleitungen darstellt, über die das Ultraschallwellen transmittierende Medium 53 der zweiten Kammer 2d zugeführt bzw. aus dieser abgeführt werden kann.

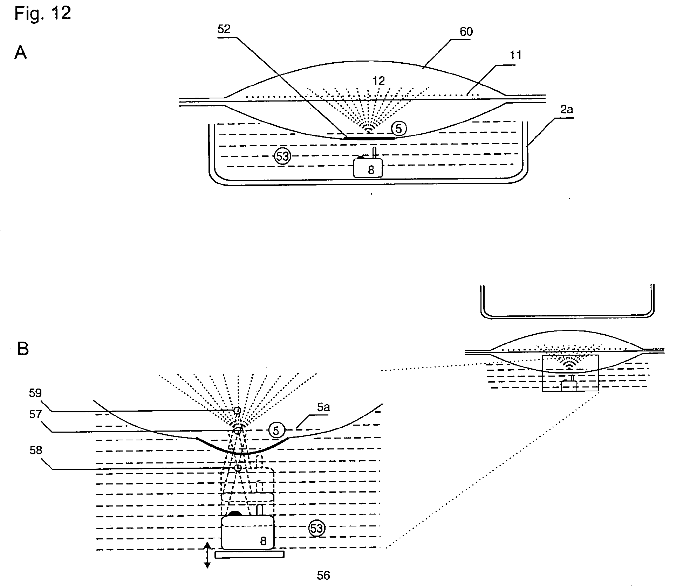

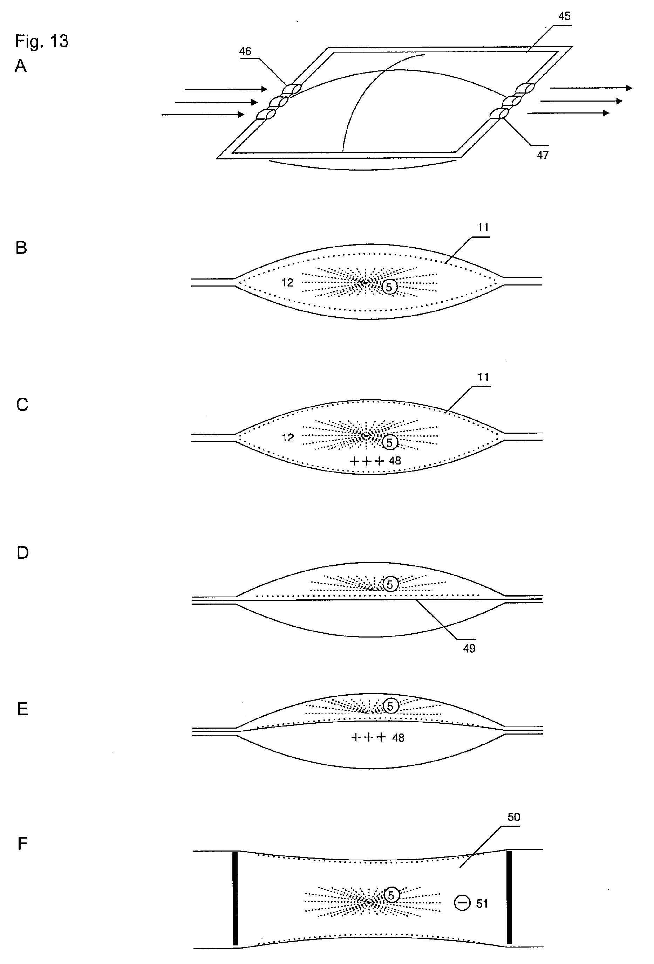

In Fig. 12A ist eine Ausführungsform der erfindungsgemäßen Vorrichtung dargestellt, in der die Kammer 2 durch ein Kissen 60 realisiert ist. Das Kissen weist in seinem Boden das Ultraschallwellen transmittierende Element 52 in Form einer Platte auf, das über seine erste Oberfläche 52a mit dem Kulturmedium 5 und mit seiner zweiten Oberfläche 52b mit dem Ultraschallwellen transmittierenden Medium 53 in direktem Kontakt steht. Das Kissen 60 ist im Zusammenhang mit Fig. 13 (dort mit der Bezugsziffer 45 versehen) im Detail beschrieben.

In der Teilabbildung Fig. 12B ist eine Ausschnittsvergrößerung des Bereiches dargestellt, in dem die Transmittierung des Ultraschalls ausgehend von dem Ultraschallgenerator 8 erfolgt, der die Ultraschallwellen in das Ultraschallwellen transmittierende Medium 53 einleitet. Über das Ultraschallwellen transmittierende Element 52, das in Form einer gewölbten Membran dargestellt ist, erfolgt die Weiterleitung in das Kulturmedium 5. Mit den Bezugsziffern 57, 58, 59 sind die Ultraschallpunkte dargestellt. Der Ultraschallbrennpunkt 57 befindet sich auf der Oberfläche 5a des Kulturmediums 5, was zu einer maximalen Zerstäubung des Kulturmediums 5 bzw. Tröpfchenbildung führt. Der Ultraschallgenerator lässt sich über eine Plattform 56 in seiner Position, vorzugsweise in vertikale Richtung, stufenlos verändern, was durch den Doppelpfeil angedeutet ist. Bei einem Absenken des Ultraschallgenerators 8 in dem Ultraschallwellen transmittierenden Medium 53 kann der Ultraschallbrennpunkt 58 in dem Ultraschallwellen transmittierenden Medium 53 positioniert werden. Bei einem Anheben des Ultraschallgenerators 8 in dem Ultraschallwellen transmittierenden Medium 53 kann der Ultraschallbrennpunkt 59 in dem Kulturmedium 5 positioniert werden.

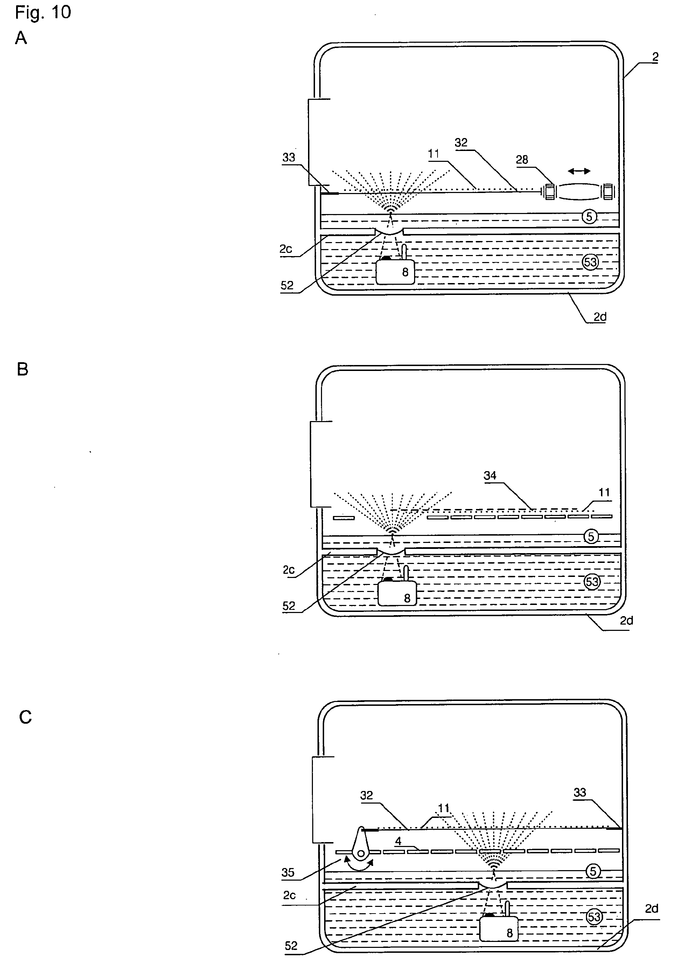

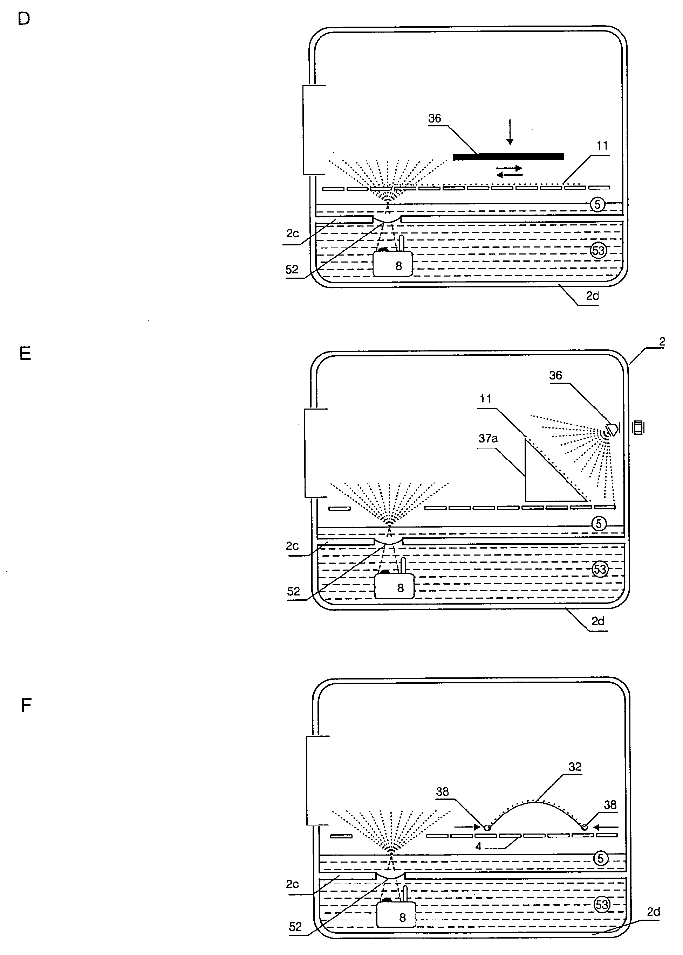

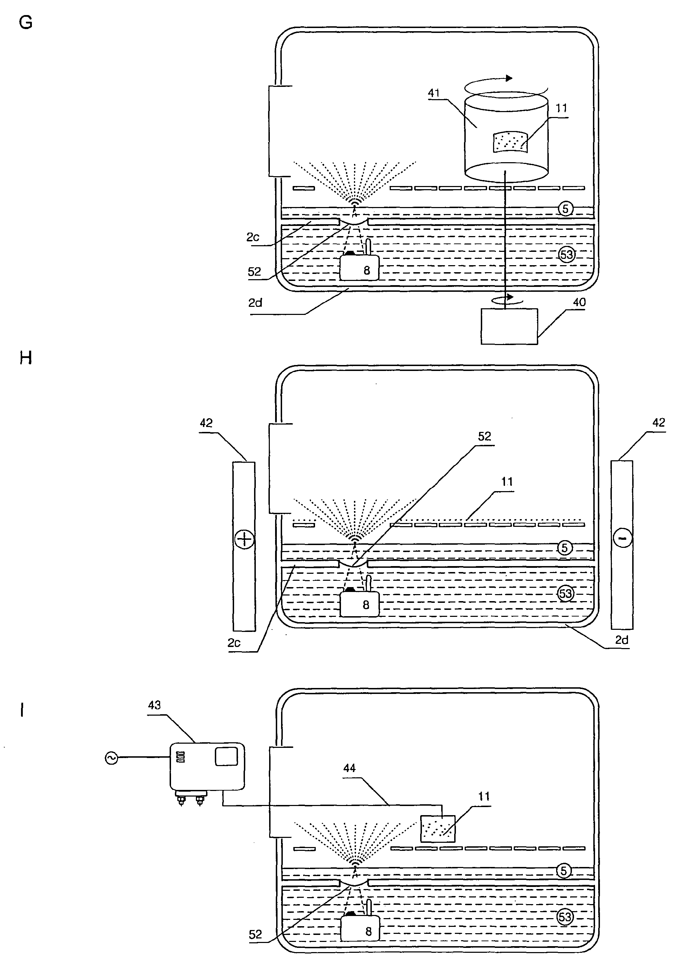

In Fig. 10 sind Ausführungsformen der erfindungsgemäßen Vorrichtung dargestellt, die Varianten der Einrichtung zur Erzeugung von physikalischen Stimuli aufweisen. Die einzelnen Elemente entsprechen denen aus den Vorrichtungen, die in der Figur 9 dargestellt sind. Die Bezugsziffern wurden, soweit möglich, beibehalten. Der Boden 2c der Kammer 2, die zweite Kammer 2d, das Ultraschallwellen transmittierende Element 52 und das Ultraschallwellen transmittierende Medium 53 sind entsprechend dargestellt. Teilabbildung Fig. 10A zeigt den pneumatischen Antrieb 28, bei dem es sich auch um einen hydraulischen Antrieb handeln kann. Teilabbildung Fig. 10B zeigt eine Variante der physikalischen Stimulierung des biologischen Materials 11, bei der kontraktile Zellen 34 vorgesehen sind, die in direktem Kontakt mit dem auf dem Träger 4 angeordneten biologischen Material 11 stehen und durch die Kontraktion die Bewegung als physikalischen Stimulus auf das Material 11 übertragen. In der Teilabbildung Fig. 10C ist als Einrichtung zur Erzeugung von physikalischen Stimuli ein mechanischer Antrieb 35 dargestellt. In Teilabbildung Fig. 10D ist ein weiterer mechanischer Antrieb 36 dargestellt, der mit dem Material 11 in Kontakt steht und auf dieses mechanischen Druck oder mechanischen Scherstress ausübt, wie dies durch die Pfeile angedeutet ist. In Teilabbildung Fig. 10E ist als Einrichtung zur Erzeugung von physikalischen Stimuli eine Gas- oder Flüssigkeitsdüse 37 dargestellt. Teilabbildung Fig. 10F zeigt eine weitere Variante der Einrichtung zur Erzeugung von physikalischen Stimuli, bei der ein weiterer mechanischer Antrieb 38 vorgesehen ist. In der Teilabbildung Fig. 10G weist die Einrichtung zur Erzeugung von physikalischen Stimuli einen Rotationsantrieb 40 auf, der in dieser Ausführungsform außerhalb der Kammer 2 und der zweiten Kammer 2d angeordnet ist. In Teilabbildung Fig. 10H ist die Einrichtung zur Erzeugung von physikalischen Stimuli durch einen Magneten 42 realisiert. In Teilabbildung Fig. 10I ist die Einrichtung zur Erzeugung von physikalischen Stimuli durch eine Spannungsgenerator 43 realisiert.

In Fig. 13 ist ein pneumatisches System in Form eines Kissens 45 gezeigt, das in Teilabbildung Fig. 13A schematisch dargestellt ist. Über Einlassöffnungen 46 kann in das Innere des Kissens 45 das Kulturmedium 5 sowie ein beliebiges Gas eingebracht werden. Das Kulturmedium 5 bzw. Gas kann über die Auslassöffnung 47 aus dem Kissen 45 wieder abgeführt werden.

Wie in Teilabbildung Fig. 13B dargestellt, kann das Innere des Kissens 45 bzw. die Innenwand des Letzteren mit dem biologischen Material 11 besiedelt werden. Die Inkubation des biologischen Materials 11 mit dem Kulturmedium erfolgt durch den in das Innere des Kissens 45 eingebrachten Nebel 12 aus Kulturmedium 5. In der Teilabbildung Fig. 13B ist das Kissen 45 in unbelastetem Zustand dargestellt, so dass kein physikalischer Stimulus auf das biologische Material 11 ausgeübt wird.

In Teilabbildung Fig. 13C wird die Zufuhrmenge von Kulturmedium 5 in Form des Nebels 12 bzw. von Gas über die Einlassöffnung erhöht und/oder die Abfuhrmenge über die Auslassöffnung 47 erniedrigt, so dass im Inneren des Kissens 45 ein Überdruck 48 (+++) entsteht. Dadurch wird das biologische Material 11 vergleichbar mit der in Fig. 9E und 10E dargestellten Variante einem Druck bzw. Scherstress und gleichzeitig durch die Verbiegung der Wandung des Kissens 45 einer Biegespannung, entsprechend der in Fig. 9F und 10F dargestellten Variante, unterworfen.

Die Teilabbildungen Fig. 13D und Fig. 13E entsprechen den Teilabbildungen 9B/10B und 9C/10C, wobei hier das Kissen 45 durch Vorsehen einer weiteren flexiblen Membran 49 im Inneren als zweikammriges Kissen ausgestaltet ist. Es versteht sich, dass durch Vorsehen einer beliebigen Anzahl von zusätzlichen, flexiblen Membranen im Inneren des Kissens 45 beliebig viele Kammern vorgesehen werden können.

In Teilabbildung Fig. 13F ist eine alternative Ausgestaltung des Kissens 50 dargestellt, bei dem durch Evakuierung von Gas im Inneren ein Unterdruck 51 (-) vorherrscht, der als physikalischer Stimulus auf das biologische Material 11 übertragen wird.

### Ausführungsbeispiel 2

Die Erfinder haben verschienene biologische Materialien mit dem erfindungsgemäßen Verfahren kultiviert bzw. generiert. Parallel wurde das gleiche biologische Material mit klassischen Kultivierungsverfahren behandelt.

Zellen der Sarkom-Zelllinie SaOs2 wurden für fünf Tage erfindungsgemäß in mittels Ultraschall erzeugtem Kulturmediumnebel und parallel auf klassische Art durch Überschichtung mit Kulturmedium, d.h. submers kultiviert. Dabei stellte sich heraus, dass beide Ansätze eine ähnliche Zelldichte hatten (Daten nicht gezeigt).

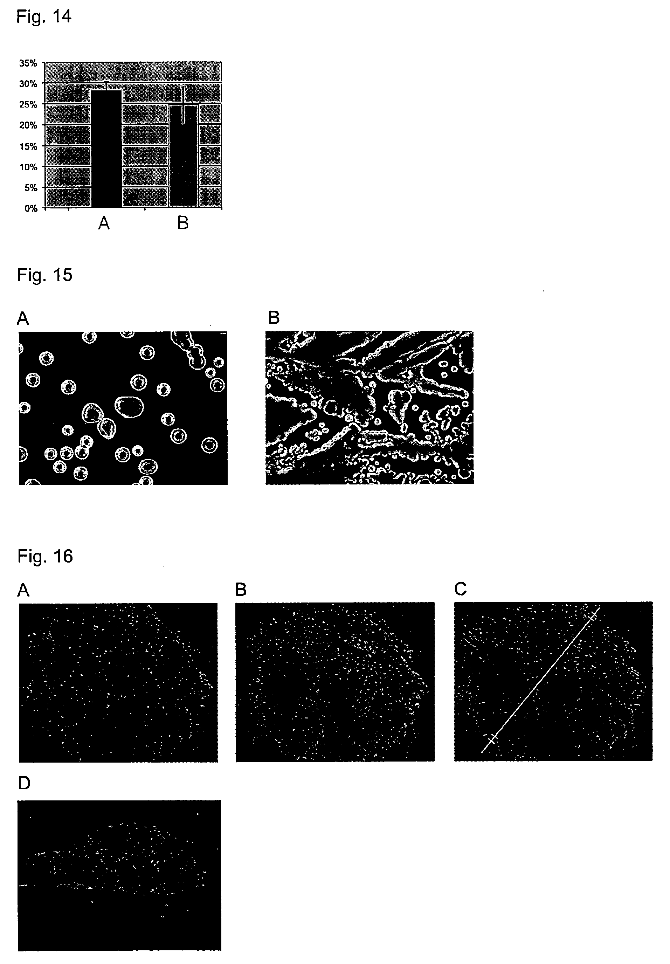

Zur Bestimmung der mitotischen Aktivität wurden die Zellen mittels Bromdesoxyuridin (BrdU) gefärbt, das sich in die DNA proliferierender Zellen einlagert. In beiden Ansätzen wurden die angefärbten Zellen ausgezählt und die Mitoserate bestimmt. Das Ergebnis ist in Fig. 14 gezeigt. Links ist das Ergebnis für die erfindungsgemäß bedampften (A) und rechts für die submers (B) behandelten Zellen dargestellt. Dabei zeigte sich, dass die erfindungsgemäß bedampften Zellen eine stärkere Proliferationsaktivität aufweisen (ca. 28 % Mitoseaktivität) als die klassisch submers kultivierten Zellen (ca. 24 %).

Die erfindungsgemäße Behandlung hat deshalb gegenüber klassischen Kultivierungsverfahren positive Auswirkungen auf die Vitalität von biologische Zellen.

In einem Folgeversuch wurde ein bakteriell kontaminiertes Kulturmedium verwendet und für 6h in die erfindungsgemäße Vorrichtung als Mediumsdampf eingebracht. Anschließend wurden Proben aus dem Dampfniederschlag entnommen. Parallel wurde das kontaminierte Medium als Flüssigkultur, d.h. unzerstäubt, unter ansonsten gleichen Bedingungen ebenfalls für 6h inkubiert. Auch aus letztem Ansatz wurden Proben entnommen. Die Proben aus beiden Ansätzen wurden für 24h auf einem Agarnährboden kultiviert. Das Bakterienwachstum in beiden Ansätzen wurde bestimmt und miteinander verglichen. Wie in Fig. 15 zu sehen ist, zeigte die Kultur aus dem Dampfniederschlags eine signifikant niedrigere Bakterienanzahl (A) als die Kultur aus der klassischen Flüssigkultur (B). Dies zeigt, dass in dem erfindungsgemäßen Verfahren Bakterien zum größten Teil getötet werden.

Bei dem erfindungsgemäßen Verfahren bzw. der erfindungsgemäßen Vorrichtung ist deshalb die Gefahr von bakteriellen Verunreinigungen des Kulturmediums deutlich gegenüber klassischen Kultivierungsverfahren reduziert.

In einem weiteren Versuch wurde untersucht, ob erfindungsgemäß behandeltes Darmgewebe seine physiologische Fuktionalität beibehält. Als Parameter hierfür wurde die Fähigkeit des embryonalen Darmgewebes herangezogen, spontan zu kontrahieren. Vom Mausembryo im Entwicklungsstadium 14 (E14) entnommenes Darmgewebe wurde für fünf Tage auf einer Millipore-Trägermembran erfindungsgemäß unter Zufuhr von Wachstumsfaktoren behandelt und anschließend das Kontraktionsvermögen untersucht. Das Ergebnis ist in Fig. 16 dargestellt. Es konnte gezeigt werden, dass die gebildete dreidimensionale Darm-Gewebekultur nach diesem Kulturzeitraum spontane physiologische Muskelkontraktionen aufwies: (A) = entspannt; (B) = kontrahiert; (C) = überlagert. Durch eine anschließende histologische Untersuchungen mit Diamin-Phenylindol-Dihydrochlorid (DAPI) und Phalloidin, die über DNA- bzw. Cytoskelett-Bindung lebende Zellen markieren, ließ sich belegen, dass die Zellen vital waren (D).

Dieses Experiment bestätigt, dass das erfindungsgemäße Verfahren positive Auswirkungen auf die Vitalität von kultivierten bzw. generierten dreidimensionalen biologischen Geweben hat, die bei klassischen Kultivierungsverfahren nicht zu beobachten sind.

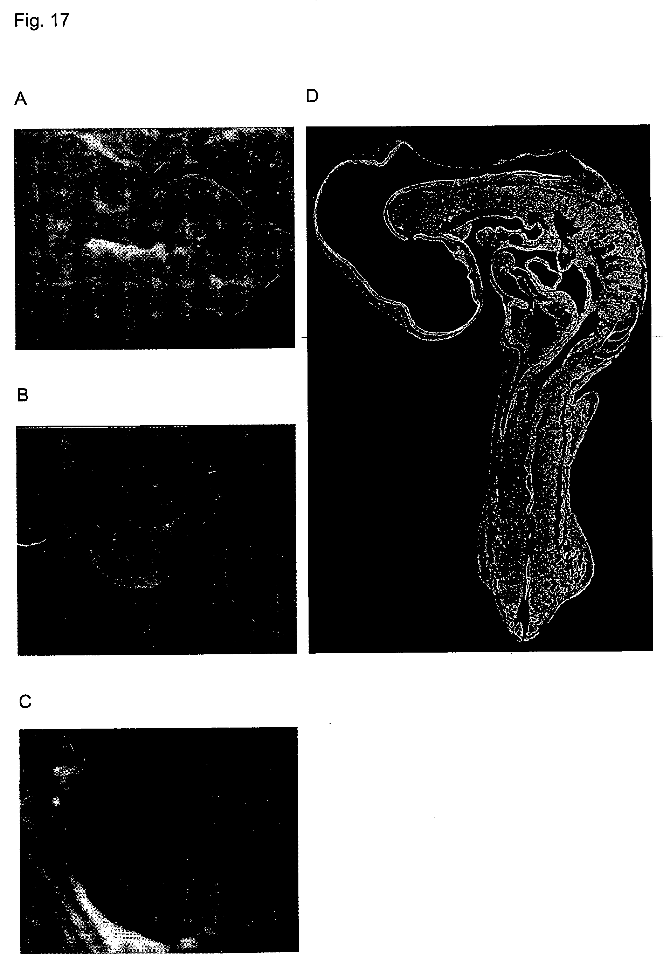

In einem weiteren Versuch wurden explantierte Hühnerembryonen im Entwicklungsstadium 12 (E12) auf einer Membran erfindungsgemäß im Mediumnebel unter Zufuhr von Wachstumsfaktoren über das Medium als physiologischer Stimulus kultiviert und die Entwicklung, d.h. die Generierung beobachtet. Das Ergebnis ist in Fig. 17 dargestellt.

Innerhalb von 48h entwickelte sich der Embryo völlig normal (A). Am Ende des Versuchs zum Stadium 18 der Embryonalentwicklung konnte beobachtet werden, dass sich ein Blutkreislauf mit strömenden Erythrozyten ausgebildet hat (C) und das Herz physiologisch kontrahierte (B). Histologische Untersuchungen bestätigten die normale Organ- und Gewebeentwicklung der ex-ovum kultivierten bzw. generierten Embryonen (D). Kontrollgruppen im konventionellen Brutschrank unter Anwendung klassischer Kultivierungsverfahren zeigten keine Vitalität.

Dieses Experiment belegt, dass es den Erfindern erstmals gelungen ist ein Verfahren und eine Vorrichtung bereitzustellen, mit dem bzw. der es unter Anwendung moderner Methoden des Tissue Engineerings gelingt, hochkomplexe biologische Einheiten, wie Embryonen, zu kultivieren bzw. zu generieren, was mit klassischen Kultivierungsverfahren bislang nicht möglich war.

## Patentansprüche

1. Vorrichtung zur Kultivierung und Generierung von biologischem Material, die Folgendes aufweist, nämlich:
- eine Einrichtung zur Erzeugung von Ultraschallwellen (8, 9, 13),
- eine ein biologisches Material (11) und ein Kulturmedium (5) aufnahmefähige Kammer (2), die
- ein Ultraschallwellen transmittierendes Element (52) aufweist, das
- eine erste Oberfläche (52a) und
- eine zweite Oberfläche (52b) aufweist,
wobei das Element (52) derart angeordnet ist, dass die erste Oberfläche (52a) mit dem Kulturmedium (5) und die zweite Oberfläche (52b) mit einem außerhalb der Kammer (2) angeordneten Ultraschallwellen transmittierenden Medium (53) in Kontakt treten kann, und
- eine Einrichtung (6, 27, 28, 34, 35, 36, 37, 38, 40, 42, 43, 60) zur Erzeugung von auf das biologische Material (11) einwirkenden physiologischen oder/und physikalischen Stimuli aufweist,
wobei die Einrichtung zur Erzeugung von Ultraschallwellen (8, 9, 13) derart außerhalb der Kammer (2) angeordnet ist, dass von dieser Ultraschallwellen in das Ultraschallwellen transmittierende Medium (53) eingeleitet und von dem Element (52) in das Kulturmedium (5) transmittiert werden können,
wobei die Einrichtung zur Erzeugung von physiologischen Stimuli durch eine Vorrichtung (6) zur Zufuhr von Wachstumsfaktoren (27a) in das Kulturmedium (5) und/oder durch eine mit dem biologischen Material (11) in Kontakt tretbare biologische Matrix oder/und eine nicht-biologische Matrix (27) realisiert wird, und die Einrichtung zur Erzeugung von physikalischen Stimuli einen pneumatischen, hydraulischen (28) oder mechanischen Antrieb (35, 36, 38) aufweist, der das biologische Material (11) in Bewegung versetzen kann.

2. Vorrichtung nach Anspruch 1, **dadurch gekennzeichnet, dass** das Ultraschallwellen transmittierende Element (52) in dem Boden (2c) der Kammer (2) angeordnet ist und ausgewählt ist aus der Gruppe bestehend aus: einer Platte oder einer Membran, bestehend aus einem Material, das ausgewählt ist aus der Gruppe bestehend aus: Keramik, Glas, Mineralien, Metall, Kunststoff und Metalllegierung.

3. Vorrichtung nach einem der vorherigen Ansprüche, **dadurch gekennzeichnet, dass** die Platte oder Membran (52) eine Dicke aufweist, die zwischen 0,005 mm und 1 cm, und vorzugsweise bei 0,025 mm liegt.

4. Vorrichtung nach einem der vorherigen Ansprüche, **dadurch gekennzeichnet, dass** das Ultraschallwellen transmittierende Medium (53) eine Flüssigkeit oder ein Gel ist.

5. Vorrichtung nach einem der vorherigen Ansprüche, **dadurch gekennzeichnet, dass** diese ferner eine das Ultraschallwellen transmittierende Medium (53) aufnahmefähige zweite Kammer (2d) aufweist, die derart ausgestaltet ist, dass diese zur zumindest teilweisen Aufnahme der Einrichtung zur Erzeugung von Ultraschallwellen (8, 9, 13) fähig ist.

6. Vorrichtung nach Anspruch 5, **dadurch gekennzeichnet, dass** die zweite Kammer (2d) derart angeordnet ist, dass zumindest eine die zweite Kammer (2d) begrenzende Wand (2c) das Ultraschallwellen transmittierende Element (52) aufweist.

7. Vorrichtung nach einem der vorherigen Ansprüche, **dadurch gekennzeichnet, dass** die Einrichtung zur Erzeugung von physikalischen Stimuli dadurch realisiert wird, dass die Kammer (2) ein Kissen (60) ist, das durch Beaufschlagung von Druck in seiner Form veränderbar ist.

8. Vorrichtung nach einem der vorherigen Ansprüche, **dadurch gekennzeichnet, dass** die Einrichtung zur Erzeugung von physikalischen Stimuli ein Mittel (36) aufweist, das in Kontakt mit dem biologischen Material (11) treten und auf dieses mechanischen Druck ausüben kann.

9. Vorrichtung nach einem der vorhergehenden Ansprüche, **dadurch gekennzeichnet, dass** die Einrichtung zur Erzeugung von physikalischen Stimuli eine Gas- oder Flüssigkeitsdüse (37) aufweist, über die auf das biologische Material (11) Gas- oder Flüssigkeitsdruck ausgeübt werden kann.

10. Vorrichtung nach einem der vorhergehenden Ansprüche, **dadurch gekennzeichnet, dass** die Einrichtung zur Erzeugung von physikalischen Stimuli einen Spannungsgenerator (43) aufweist, der dem biologischen Material (2) elektrische Impulse zuführen kann.

11. Vorrichtung nach einem der vorhergehenden Ansprüche, **dadurch gekennzeichnet, dass** die Einrichtung zur Erzeugung von physikalischen Stimuli einen Magneten (42) aufweist, der das biologische Material (11) einem Magnetfeld aussetzen kann.

12. Vorrichtung nach einem der vorhergehenden Ansprüche, **dadurch gekennzeichnet, dass** die Einrichtung zur Erzeugung von physikalischen Stimuli kontraktile biologische Zellen (34) aufweist, die mit dem biologischen Material (11) in Kontakt treten können.

13. Verfahren zum Kultivieren und Generieren von biologischem Material, ausgenommen menschliche Lebewesen, menschliche Embryonen und menschliche embryonale Stammzellen, das folgende Schritte aufweist:
(a) Bereitstellen von biologischem Material (11) und Kulturmedium (5) in einer Kammer (2), die
- ein Ultraschallwellen transmittierendes Element (52) aufweist, das
- eine erste Oberfläche (52a) und
- eine zweite Oberfläche (52b) aufweist,
- wobei das Element (52) derart angeordnet ist, dass die erste Oberfläche (52a) mit dem Kulturmedium (5) und die zweite Oberfläche (52b) mit einem außerhalb der Kammer (2) angeordneten Ultraschallwellen transmittierenden Medium (53) in Kontakt treten kann,
(b) Einleiten von Ultraschallwellen in das Ultraschallwellen transmittierende Medium (53) derart, dass diese von dem Element (52) in das Kulturmedium (5) transmittiert werden.

14. Verfahren nach Anspruch 13, **dadurch gekennzeichnet, dass** in Schritt (b) die Ultraschallwellen derart in das transmittierende Medium (53) eingeleitet werden, dass ein Ultraschallbrennpunkt (57, 58, 59) in dem Kulturmedium (5) oder in dem Ultraschallwellen transmittierenden Medium (53) entsteht.

15. Verfahren nach Anspruch 14, **dadurch gekennzeichnet, dass** in Schritt (b) die Ultraschallwellen derart in das Kulturmedium (5) eingeleitet werden, dass der Ultraschallbrennpunkt (57) mit der Oberfläche (5a) des Kulturmediums (5) zusammen fällt.

16. Verfahren nach einem der vorherigen Ansprüche, **dadurch gekennzeichnet, dass** in einem weiteren Schritt (c) eine physiologische oder/und physikalische Stimulierung des biologischen Materials (11) erfolgt.

## Claims

1. A device for the cultivation and generation of biological material which comprises the following, namely:
- a unit for generating ultrasonic waves (8, 9, 13),
- a chamber (2) able to receive a biological material (11) and a culture medium (5), comprising
- an element (52) which transmits ultrasonic waves, comprising
- a first surface (52a) and
- a second surface (52b),
where the element (52) is disposed in such a way that the first surface (52a) can make contact with the culture medium (5) and the second surface (52b) can make contact with a medium (53) which transmits ultrasonic waves and is disposed outside the chamber (2), and
- a unit (6, 27, 28, 34, 35, 36, 37, 38, 40, 42, 43, 60) for generating physiological and/or physical stimuli acting on the biological material (11),
where the unit for generating ultrasonic waves (8, 9, 13) is disposed outside the chamber (2) in such a way that from these ultrasonic waves can be introduced into the medium (53) which transmits ultrasonic waves, and be transmitted by the element (52) into the culture medium (5),
where the unit for generating physiological stimuli is achieved by a device (6) for delivering growth factors (27a) into the culture medium (5), and/or by a biological matrix which can make contact with the biological material (11), or/and by a non-biological matrix (27), and where the unit for generating physical stimuli comprises a pneumatic or hydraulic or mechanical drive (35, 36, 38 which is able to set the biological material (11) in motion.

2. The device of claim 1, **characterized in that** the element (52) which transmits ultrasonic waves is disposed in the base (2c) of the chamber (2), and is selected from the group consisting of: a plate or a diaphragm which consists of a material which is selected from the group consisting of: ceramic, glass, minerals, metal, plastics and metal alloy.

3. The device of any of the preceding claims, **characterized in that** the plate or diaphragm (52) has a thickness which is between 0.005 mm and 1 cm, and is preferably 0.025 mm.

4. The device of any of the preceding claims, **characterized in that** the medium (53) which transmits ultrasonic waves is a liquid or a gel.

5. The device as claimed in any of the preceding claims, **characterized in that** it further comprises a second chamber (2d) which is designed so that it is capable of at least partial reception of the unit for generating ultrasonic waves (8, 9, 13).

6. The device of claim 5, **characterized in that** the second chamber (2d) is disposed so that at least one boundary wall (2c) of the second chamber (2d) comprises the element (52) which transmits ultrasonic waves.

7. The device of any of the preceding claims, **characterized in that** the unit for generating physical stimuli is achieved by the chamber (2) being a cushion (60) whose shape can be altered by applying pressure.

8. The device of any of the preceding claims, **characterized in that** the unit for generating physical stimuli comprises means (36) able to make contact with the biological material (11) and exert mechanical pressure thereon.

9. The device of any of the preceding claims, **characterized in that** the unit for generating physical stimuli comprises a gas or liquid nozzle (37) via which gas or liquid pressure can be exerted on the biological material (11).

10. The device of any of the preceding claims, **characterized in that** the unit for generating physical stimuli comprises a voltage generator (43) able to deliver electrical pulses to the biological material (2).

11. The device of any of the preceding claims, **characterized in that** the unit for generating physical stimuli comprises a magnet (42) able to expose the biological material (11) to a magnetic field.

12. The device of any of the preceding claims, **characterized in that** the unit for generating physical stimuli comprises contractile biological cells (34) able to make contact with the biological material (11).

13. A method for the cultivation and generation of biological material, excluding human beings, human embryos, and human embryonic stem cells, which comprises the following steps:
(a) provision of biological material (11) and culture medium (5) in a chamber (2) which comprises
- an element (52) which transmits ultrasonic waves and which has
- a first surface (52a) and
- a second surface (52b),
- where the element (52) is disposed in such a way that the first surface (52a) can make contact with the culture medium (5) and the second surface (52b) can make contact with a medium (53) which transmits ultrasonic waves and is disposed outside the chamber (2);
(b) introduction of ultrasonic waves into the medium (53) which transmits ultrasonic waves in such a way that they are transmitted by the element (52) into the culture medium (5).

14. The method of claim 13, **characterized in that** the ultrasonic waves are introduced into the transmitting medium (53) in step (b) in such a way that an ultrasound focus (57, 58, 59) results in the culture medium (5) or in the medium (53) which transmits ultrasonic waves.

15. The method of claim 14, **characterized in that** the ultrasonic waves are introduced into the culture medium (5) in step (b) in such a way that the ultrasound focus (57) coincides with the surface (5a) of the culture medium (5).

16. The method as claimed in any of the preceding claims, **characterized in that** a physiological or/and physical stimulation of the biological material (11) takes place in a further step (c).

## Revendications

1. Dispositif de culture et de production d'un matériau biologique, qui présente les éléments suivants, à savoir :
- un système servant à générer des ondes ultrasonores (8, 9, 13),
- une chambre (2) apte à recevoir un matériau biologique (11) et un milieu de culture (5), laquelle
- - présente un élément (52) transmettant des ondes ultrasonores, qui présente
- - - une première surface (52a) et
- - - une deuxième surface (52b),
sachant que l'élément (52) est disposé de telle manière que la première surface (52a) peut entrer en contact avec le milieu de culture (5) et que la deuxième surface (52b) peut entrer en contact avec un milieu (53) transmettant des ondes ultrasonores, disposé à l'extérieur de la chambre (2), et
- un système (6, 27, 28, 34, 35, 36, 37, 38, 40, 42, 43, 60) servant à générer des stimuli physiologiques et/ou physiques agissant sur le matériau biologique (11),
sachant que le système servant à générer des ondes ultrasonores (8, 9, 13) est disposé à l'extérieur de la chambre (2) de telle manière que des ondes ultrasonores peuvent être introduites dans le milieu (53) transmettant des ondes ultrasonores par ladite chambre et peuvent être transmises dans le milieu de culture (5) par l'élément (52),
sachant que le système servant à générer des stimuli physiologiques est réalisé par un dispositif (6) servant à amener des facteurs de croissance (27a) dans le milieu de culture (5) et/ou par une matrice biologique pouvant entrer en contact avec le matériau biologique (11) et/ou par une matrice non biologique (27), et que le système servant à générer des stimuli physiques présente un entraînement (35, 36, 38) pneumatique, hydraulique (28) ou mécanique, qui peut amener le matériau biologique (11) en mouvement.

2. Dispositif selon la revendication 1, **caractérisé en ce que** l'élément (52) transmettant des ondes ultrasonores est disposé dans le fond (2c) de la chambre (2) et est choisi parmi le groupe constitué : d'une plaque ou d'une membrane, constituée d'un matériau qui est choisi parmi le groupe constitué de : céramique, verre, minéraux, métal, matière plastique et alliage de métal.

3. Dispositif selon l'une quelconque des revendications précédentes, **caractérisé en ce que** la plaque ou la membrane (52) présente une épaisseur, qui est comprise entre 0,005 mm et 1 cm, qui de préférence est de l'ordre de 0,025 mm.

4. Dispositif selon l'une quelconque des revendications précédentes, **caractérisé en ce que** le milieu (53) transmettant des ondes ultrasonores est un liquide ou un gel.

5. Dispositif selon l'une quelconque des revendications précédentes, **caractérisé en ce que** ledit dispositif présente en outre une deuxième chambre (2d) apte à recevoir le milieu (53) transmettant des ondes ultrasonores, laquelle chambre est configurée de telle manière qu'elle est apte à recevoir au moins en partie le système servant à générer des ondes ultrasonores (8, 9, 13).

6. Dispositif selon la revendication 5, **caractérisé en ce que** la deuxième chambre (2d) est disposée de telle manière qu'au moins une paroi (2c) délimitant la deuxième chambre (2d) présente l'élément (52) transmettant des ondes ultrasonores.

7. Dispositif selon l'une quelconque des revendications précédentes, **caractérisé en ce que** le système servant à générer des stimuli physiques est réalisé **en ce que** la chambre (2) est un coussin (60), dont la forme peut être modifiée sous l'action de la contrainte exercée par la pression.

8. Dispositif selon l'une quelconque des revendications précédentes, **caractérisé en ce que** le système servant à générer des stimuli physiques présente un moyen (36), qui peut entrer en contact avec le matériau biologique (11) et qui peut exercer sur ce dernier une pression mécanique.

9. Dispositif selon l'une quelconque des revendications précédentes, **caractérisé en ce que** le système servant à générer des stimuli physiques présente une buse à gaz ou à liquide (37), par l'intermédiaire de laquelle une pression de gaz ou de liquide peut être exercée sur le matériau biologique (11).

10. Dispositif selon l'une quelconque des revendications précédentes, **caractérisé en ce que** le système servant à générer des stimuli physiques présente un générateur de tension (43), qui peut amener au matériau biologique (2) des impulsions électriques.

11. Dispositif selon l'une quelconque des revendications précédentes, **caractérisé en ce que** le système servant à générer des stimuli physiques présente un aimant (42), qui peut soumettre le matériau biologique (11) à un champ magnétique.

12. Dispositif selon l'une quelconque des revendications précédentes, **caractérisé en ce que** le système servant à générer des stimuli physiques présente des cellules (34) biologiques contractiles, qui peuvent entrer en contact avec le matériau biologique (11).

13. Procédé de culture et de production d'un matériau biologique, à l'exception d'êtres vivants humains, d'embryons humains et de cellules souches embryonnaires humaines, lequel procédé comprend les étapes suivantes consistant à :
(a) fournir un matériau biologique (11) et un milieu de culture (5) dans une chambre (2), qui
- présente un élément (52) transmettant des ondes ultrasonores, lequel élément présente
- - une première surface (52a) et
- - une deuxième surface (52b),
- sachant que l'élément (52) est disposé de telle manière que la première surface (52a) peut entrer en contact avec le milieu de culture (5) et que la deuxième surface (52b) peut entrer en contact avec le milieu (53) transmettant des ondes ultrasonores, disposé à l'extérieur de la chambre (2),
(b) introduire des ondes ultrasonores dans le milieu (53) transmettant des ondes ultrasonores de telle manière que ces dernières sont transmises par l'élément (52) dans le milieu de culture(5).

14. Procédé selon la revendication 13, **caractérisé en ce que**, à l'étape (b), les ondes ultrasonores sont introduites dans le milieu (53) de transmission de telle manière qu'un point de convergence des ondes ultrasonores (57, 58, 59) apparaît dans le milieu de culture (5) ou dans le milieu (53) transmettant des ondes ultrasonores.

15. Procédé selon la revendication 14, **caractérisé en ce que**, à l'étape (b), les ondes ultrasonores sont introduites dans le milieu de culture (5) de telle manière que le point de convergence des ondes ultrasonores (57) coïncide avec la surface (5a) du milieu de culture (5).

16. Procédé selon l'une quelconque des revendications précédentes, **caractérisé en ce que**, lors d'une autre étape (c), une stimulation physiologique et/ou physique du matériau biologique (11) a lieu.
